(19) Europäisches Patentamt
European Patent Office
Office européen des brevets

(11) Veröffentlichungsnummer: **0 398 287 A1**

(12) **EUROPÄISCHE PATENTANMELDUNG**

(21) Anmeldenummer: **90109234.6**

(22) Anmeldetag: **16.05.90**

(51) Int. Cl.$^5$: **A61K 9/08, A61K 9/48**

(30) Priorität: **16.05.89 US 352269**

(43) Veröffentlichungstag der Anmeldung: **22.11.90 Patentblatt 90/47**

(84) Benannte Vertragsstaaten: **AT BE CH DE DK ES FR GB GR IT LI LU NL SE**

(71) Anmelder: **MEDICE Chem.-Pharm. Fabrik Pütter GmbH & Co. KG**
**Kuhloweg 37-39**
**D-5860 Iserlohn/Westfalen(DE)**

(72) Erfinder: **Paradies Henrich H. Prof. Dr. Dr.**
**Goerresstr.38**
**5860 Iserlohn(DE)**
Erfinder: **Hanna, Samir B. Prof.Ph.D.**
**Mann Court Route 4**
**Rolla, Missouri MO 65401(US)**
Erfinder: **Schneider, Bernd**
**Kuhloweg 36**
**5860 Iserlohn(DE)**

(74) Vertreter: **Reinhard, Skuhra, Weise**
**Friedrichstrasse 31**
**D-8000 München 40(DE)**

(54) **Pharmazeutische Zubereitung.**

(57) Die Erfindung betrifft eine pharmazeutische Zubereitung, bestehend aus einem oder mehreren Wirkstoffen und einem Träger, der im Bereich zwischen 20° und 80° C erstarrt und wasserlöslich ist,
**dadurch gekennzeichnet,**
daß sie eine isotrope Lösung ist, wobei

a) der Wirkstoff in dem Träger monomolekular oder als Ion gelöst ist,

b) der Wirkstoff in seiner nativen Konformation und/oder seiner biologisch aktiven chiralen (enantiomeren) Konformation vorliegt,

c) der Wirkstoff einen Molenbruch von 0,001 bis 0,67 bei 37° C aufweist,

d) der Träger bei Körpertemperatur schmelzflüssig, phaseneinheitlich und isotrop ist,

e) die isotrope Lösung, bestehend aus Träger und Wirkstoff, bei Raumtemperatur erstarrt,

f) die erstarrte Lösung kristallin oder nicht-kristallin ist oder sie den Wirkstoff kristallin enthält oder sie den Wirkstoff auskristallisieren kann,

g) die monomolekulare oder ionische Lösung einen osmotischen Druck hat und eine molare Gefrierpunkterniedrigung bewirkt,

h) der gelöste Wirkstoff innerhalb des Polymerelektrolyten einen temperaturabhängigen Diffusionskoeffizienten hat und eine temperaturabhängige spezifische Leitfähigkeit hat sowie ein Verfahren zur Herstellung dieser pharmazeutischen Zubereitung.

EP 0 398 287 A1

## Pharmazeutische Zubereitung

Die vorliegende Erfindung betrifft eine pharmazeutische Zubereitung und ein Verfahren zu ihrer Herstellung.

Pharmazeutische Zubereitungen bestehen im wesentlichen aus einem oder mehreren Wirkstoffen (Heilstoffen) und einem Träger.

Nicht-wäßrige Lösungen, Emulsionen und auch Mikro-Emulsionen als mögliche pharmazeutische Träger bzw. pharmazeutische Formulierungen sind hinreichend bekannt (siehe z.B. M.J. Schick et al.: "Emulsions and emulsion technology," Vol. 6, Surfactant Science Series, II, Chapter 13," Cosmetic Emul sions," 1974, 729-730, ed. J. Lissant Marcel Dekker, Inc., N.Y., U.S.A., M.J. Schick in "Nonionic Surfactants, Physical Chemistry, 1988, Marcel Dekker, Inc., N.Y. and Basel). So behandeln speziell die meisten bekannten Veröffentlichungen Emulsionen, u.a. mit öl-löslichen Matrizen, kombiniert mit Wachs oder Pasten, mit und ohne wäßrigen Systemen (öl-lösliche und wasser-lösliche Matrizen), die in Kapseln, speziell in Weichgelatinekapseln, eingefüllt werden können. Die wasser-löslichen inerten Matrix-Substanzen sind meistens Polyethylenglykole (PEG) mit kleinen oder mittleren Molekulargewichten oder Mischungen aus niedrigen und hohen Molekulargewichten, z.B. PEG 300 oder 400, PEG 1500, PEG 4000 oder PEG 6000. Öl-lösliche Matrizen beinhalten pflanzliche oder tierische Öle, welche - wenn gewünscht mit Wachs oder Fetten als Verfestiger, etc. - auch in Zusammenhang mit Mineralölen in Weichgelatinekapseln eingesetzt werden können.

Zu diesen wasserlöslichen, inerten Matrixsubstanzen gehört auch das Polyethylenglykol-660-12-hydroxystearat mit den gleichen physikalisch-chemischen Eigenschaften, wie sie für Polyethylenglykole (PEG) oder Polyethylenoxide (PEO) beschrieben worden sind.

Alle diese Beschreibungen, insbesondere die der Europäischen Patentschrift vom 27.09.1983 (Anmeldenummer 83109633.4, "Anhydrous emulsions and the use thereof"), behandeln weder homogene, flüssige Lösungen von inertem Stoff und pharmazeutischem Wirkstoff noch feste Lösungen. Die EP 83109833.4 beschreibt eine pharmazeutische Formulierung auf der Basis einer Emulsion, die bei 37° C schmilzt. Dabei ist es notwendig, bei dieser Art von Emulsionen zur Füllung von Hartgelatinekapseln die thixotropen Prozesse zu beachten, um die Kapseln am Undichtsein zu hindern.

Liegt z.B. ein Arzneimittelwirkstoff in Kristallform oder als Pulver vor, so kann man die Kristalle oder das Pulver als solche in die Hartgelatinekapseln einbringen. Dies ist tech nisch aber schwierig und läßt sich in der Regel nicht ohne Hilfsstoffe und ohne aufwendige Bearbeitung durchführen; denn der Wirkstoff muß in einer ganz genauen Dosismenge in die Kapsel eingebracht werden. Dieses Problem kann man dadurch lösen, daß man den Wirkstoff als Schmelze in die Kapsel einbringt. Da aber beim Erstarren dieser Schmelze der Wirkstoff dann in relativ kompakter Form in der Hartgelatinekapsel enthalten ist, erfolgt die Freisetzung des Wirkstoffes aufgrund der verringerten Oberfläche nur verzögert.

Während bei den Weichgelatinekapseln gemäß dem Stand der Technik die Wechselwirkungen zwischen Wirkstoff und Gelatine als auch inerter Matrix mit Gelatine zu beachten sind, ist dies bei Hartgelatinekapseln nicht von großer Bedeutung (siehe z.B. Armstrong, N.A., Jones, K.C., Dugh, W.K.L., J. Pharm. Pharmacol., 1984, 36, 361-365; Horn, F.S., Veresk, S.A., Miskel, J.J., J. Pharm Sci., 1973, 62, 1001-1006). Dennoch sind Wechselwirkungen verschiedenster Art sowohl der inerten Matrix wie auch der pharmazeutischen Wirkstoffe mit der Hartgelatinekapsel nicht auszuschließen (vgl. C.M. Ofner III, and H. Schott, J. Pharm. Sci., 76, 715-723, 1987; C.M. Ofner III, and H. Schott, J. Pharm. Sci., 75, 790-796, 1985).

Soweit der Stand der Technik Dispersionen betrifft, sind deren Nachteile bekannt, nämlich unregelmäßige Konzentrationsverteilung, Verteilung (Partitioning) zwischen verschiedenen Phasen, Löslichkeitsprobleme, unkontrollierte Freisetzung, Abhängigkeit von thixotropen Prozessen etc..

Aus der europäischen Patentanmeldung 78101259.6 ist bereits ein Verfahren zur Herstellung von pharmazeutischen Präparaten in Hartkapselform bekannt, wobei man einen den Wirkstoff enthaltenden flüssigen Träger, der als wasserlösliche Schmelzmasse mit einem Erstarrungspunkt im Bereich zwischen 30 und 60° C oder als thixotropes Gel vorliegt, in eine zur Verabreichung als Dosiereinheit geeignete starre Hülle eindosiert. Hierzu wird eine spezielle Kapselfüllmaschine verwendet. Nach dieser europäischen Patentanmeldung kann der den Wirkstoff enthaltende flüssige Träger beispielsweise ein thixotropes Gel sein, das sich während des Abfüllvorgangs wie eine Flüssigkeit, aber innerhalb der Kapselhülle wie ein Feststoff verhält, oder er kann eine wasserlösliche Schmelzmasse sein, die dann beim Abkühlen auf Raumtemperatur zu einer festen Lösung, einer festen Dispersion oder einem eutektischen Gemisch oder auch einem Gemisch dieser Formen erstarrt. Z.B. kann sich ein Arzneimittel teilweise in einem geschmolzenen Träger auflösen, so daß beim Erstarren ein Gemisch aus fester Lösung und fester Dispersion entsteht. Mit diesem Verfahren soll die Aufgabe gelöst werden, ein Arzneimittel bereitzustellen, das in feinverteilter

Form vorliegt und das leicht und schnell resorbiert wird.

Die resultierende pharmazeutische Zubereitung weist aber den wesentlichen Nachteil auf, daß sie das Erfordernis, in feinverteilter Form vorzuliegen, nicht erfüllt, weil die angeblich hergestellten festen Lösungen in Wahrheit keine festen Lösungen, sondern Dispersionen sind, für welche die bereits weiter oben angegebenen Nachteile gelten.

Außerdem weisen sie den Nachteil auf, daß sie weder isotrop sind noch eine einheitliche Phase bilden. Darüber hinaus stellen sie keine echten Lösungen im Sinne der Elektrolyttheorie dar, d.h., es bilden sich keine elektro-aktiven Partikel aus, z.B. Kationen oder Anionen der entsprechenden pharmazeutischen Wirkstoffe, wobe das Lösungsmittel (Matrix) nicht als ionen-leitfähig angesehen wird (Ionen bzw. Dissoziations-Gleichgewicht).

Spezielle, Wirkstoff-immanente Schwierigkeiten können für reine, enantiomere Stoffe auftreten, wie z.B. Razemisierung, Umwandlung in andere (polymorphe) Kristallformen, Zerfall der enantiomeren Substanz und andere unerwünschte in vivo und in vitro Freisetzungseigenschaften.

Der wesentliche Nachteil dieses Verfahrens liegt jedoch auch darin, daß eine Razematisierung eines chiralen Wirkstoffes, der entweder ein oder mehrere chirale Zentren aufweist, wie z.B. (S)-oder (R)-Ibuprofen oder Prostaglandine, z.B. $PGE_{2\alpha}$ oder $PGE_2$ , eintreten kann, da es sich nicht um echte, physikalisch-chemisch definierte Lösungen handelt. Diese vorne genannten Offenbarungen zeigen außerdem nicht, inwieweit Razematisierungen bzw. eine Trennung in Enantiomere auftreten, da nur razemische Wirkstoffe beschrieben worden sind. Detaillierte Untersuchungen zu diesem sehr wichtigen Problemkreis liegen nicht vor. Dies gilt insbesondere auch für polymorphe, kristalline Wirkstoffe, die bei Vorliegen in disperser Lösung sich nicht nur molekular verteilen können, sondern auch technisch-physikalische Schwierigkeiten verursachen, so daß diese pharmazeutischen Fromulierungen entweder nicht erstarren, sondern flüssig bis zäh-viskos bleiben oder beim Erstarren Gele bzw. glasartige Strukturen ausbilden. Ein weiterer Nachteil besteht darin, daß die vorliegenden Dispersionen je nach physikalisch-chemischer Beschaffenheit unkontrollierbare Mengen von pharmazeutischen Wirkstoffen enthalten, wie von uns experimentell festgestellt werden konnte. Diese Dispersionen bestehen hauptsächlich aus kolloidalen Teilchen von pharmazeutischen Wirkstoffen verschiedener Aggregationszahl und Größe (Cluster) und weniger aus Aggregaten oder komplexen Gebilden aus Matrix und pharmazeutischem Wirkstoff, so daß physikalisch keine Einheitlichkeit, wie sie bei einer Lösung anzutreffen ist, besteht. Daraus resultiert technisch, daß eine genaue und zuverlässige Dosierung pharmazeutischer Wirkstoffe nicht gegeben ist. Dies ist umsc bedeutungsvoller beim Einsatz von pharmazeutischen Wirkstoffen in Form von Salzen, z.B. den Alkali- oder Erdalkalisalzen von Wirkstoffen mit Karboxylgruppen oder alkoholischen (primären) Gruppen (Enolformen), den N-(2-Hydroxyethyl) piperazinium Salzen, N-Methyl-glukosammonium, Lysin- und Arginin-Salzen. Diese salzartigen Verbindungen bilden u.a. nach dem vorne genannten Verfahren Diffusions-Barrieren aus, so daß auch u.a. Entmischungsphänomene im mikroskopischen Bereich beobachtbar wer den. Dies wiederum bedingt ungünstige Freisetzungen von pharmazeutischen Wirkstoffen in vitro und in vivo.

Eine Aufgabe der Erfindung ist es daher, eine neue pharmazeutische Zubereitung zur Verfügung zu stellen, in welcher der oder die Wirkstoffe nach Verabreichung schnell freigegeben und resorbiert werden.

Diese Aufgabe wird erfindungsgemäß dadurch gelöst, daß eine pharmazeutische Zubereitung, bestehend aus einem oder mehreren Wirkstoffen und einem Träger, der im Bereich zwischen 20° C und 80° C erstarrt und wasserlöslich ist, bereitgestellt wird, der dadurch gekennzeichnet ist, daß sie eine isotope Lösung ist, wobei

a) der Wirkstoff in dem Träger monomolekular oder als Ion gelöst ist,

b) der Wirkstoff in seiner nativen Konformation und/oder seiner biologisch aktiven chiralen (enantiomeren) Konformation vorliegt,

c) der Wirkstoff einen Molenbruch von 0,001 bis 0,67 bei 37° C aufweist,

d) der Träger bei Körpertemperatur schmelzflüssig, phaseneinheitlich und isotrop ist,

e) die isotrope Lösung, bestehend aus Träger und Wirkstoff, bei Raumtemperatur erstarrt,

f) die erstarrte Lösung kristallin oder nicht-kristallin ist oder den Wirkstoff kristallin enthält oder auskristallisieren kann,

g) die monomolekulare oder ionische Lösung einen osmotischen Druck hat und eine molare Gefrierpunkterniedrigung bewirkt,

h) der gelöste Wirkstoff innerhalb des Polymerelektrolyten einen temperaturabhängigen Diffusionskoeffizienten hat und eine temperaturabhängige spezifische Leitfähigkeit hat.

Die erfindungsgemäße pharmazeutische Zubereitung besteht bevorzugt aus 50 - 99,9990 Gew.% Träger und 0,0010 - 50 Gew.% Wirkstoff oder Wirkstoffgemisch.

Weiterhin bevorzugt sind 50 - 55 Gew.% Träger und 45 - 50 Gew.% Wirkstoff oder Wirkstoffgemisch, 60 - 85 Gew.% Träger und 15 - 40 Gew.% Wirkstoff oder Wirkstoffgemisch, 70 - 80 Gew.% Träger und 20

- 30 Gew.% Wirkstoff oder Wirkstoffgemisch, 75 - 85 Gew.% Träger und 15 - 25 Gew.% Wirkstoff oder Wirkstoffgemisch, 90 - 99,99 Gew.% Träger und 0,01 - 10 Gew.% Wirkstoff oder Wirkstoffgemisch.

Weiterhin bevorzugt besteht die erfindungsgemäße pharmazeutische Zubereitung aus 50 Gew.% Träger und 50 Gew.% S-Ibuprofen oder 99,98 Gew.% Träger und 0,02 Gew.% Dinoprost oder 99,9975 Gew.% Träger und 0,0025 Gew.% Arg-Vasopressin.

Der Träger ist bevorzugt wasserfrei. Die pharmazeutische Zubereitung kann jedoch zusätzlich bis zu ca. 1 Gew.-% Wasser oder Ethanol, bezogen auf die gesamte pharmazeutische Zubereitung, enthalten.

Erfindungsgemäß bevorzugt wird als Träger ein Polymer mit einem Molekulargewicht von 200 - 10.000. Insbesondere bevorzugt ist der Träger ein Polyethylenglykol, ein Polyoxyethylenglykol, ein Polypropylenglykol, ein Polyoxyethylenoxid, ein Polypropylenoxid, ein Paraffinester, ein Isopropylpalmitat, ein Isopropylmyristat, ein Myristylalkohol, Palmitinalkohol und/oder ein Stearylalkohol.

Erfindungsgemäß bevorzugt weist der Wirkstoff einen Molenbruch von 0,05 bis 0,25 auf.

Ein weiterer Vorteil dieser Erfindung besteht in der Schaffung einer pharmazeutischen Zubereitung, in der die pharmazeutischen Wirkstoffe u.a. so gelöst werden, daß sie nach Abkühlen bzw. Erstarren kristallin oder amorph mit einer Substanz-immanenten Nahordnung in Erscheinung treten und die gleiche native, biologisch-aktive Konformation aufweisen, wie sie medizinisch-therapeutisch gefordert wird.

Ein weiterer Vorteil gegenüber dem Stand der Technik besteht darin, daß ein bzw. mehrere geeignete Polymere (Matrix = Träger) bereitgestellt werden, welche zur Gruppe der Polymer-Elektrolyte gehören, welche Lösungsmittetl (flüssig)-frei, aber ionen-leitfähig und in der Lage sind, feste Lösungen von dissoziierbaren, pharmazeutischen Wirkstoffen nach Abkühlen auf Raumtemperatur zu bilden.

Ein weiterer Vorteil liegt darin, daß diese Polymer-Elektrolyte neutrale bzw. dissoziierbare pharmazeutische Wirkstoffe als Monomere lösen können, so daß sie in der Schmelze (flüssig) in das Netzwerk des Polymeren (Lösungsmittel) diffundieren können. Um diese Diffusion zu beschleunigen ist die Matrix (Lösungsmittel) in flüssiger Form vorzulegen, so daß nach Erstarren der Schmelze (Flüssigkeit) der pharmazeutische Wirkstoff, insbesondere die Enantiomeren mit hoher optischer Aktivität, unter Beibehaltung ihrer absoluten Konformation (ohne Razematisierung) mono-molekular, eventuell als Kation oder Anion, vorliegen.

Ein weiterer Vorteil besteht darin, daß sowohl die Phasenstruktur der Matrix (Lösungsmittel, $n_1$) wie auch der pharmazeutische Wirkstoff nach Eintrag der Wirkstoffe (Gelöstes $n_2$) im flüssigen Zustand (Schmelze) als auch nach Abkühlung auf Raumtemperatur (fest) erhalten bleibt und sowohl in flüssigem sowie auch im festen Zustand keine Phasentrennung (z.B. Entmischung) eintritt, so daß ein isotropes Ein-Phasen-System entsteht ($n = n_2/n_1 + n_2$, mit n = Molenbruch).

Erfindungsgemäß gehören die hier angewandten Polymere (Matrix = Träger) zur Gruppe der Polymer-Elektrolyte (siehe M.A. Ratner, D.F. Schriver, Chem. Rev. 1988, 88, 109; M.B. Armand, Annu, Rev. mater. Sci. 1986, 16, 245) wie z.B. Polymere, welche Polyoxyethylenoxid (PEO)-Gruppen enthalten. Diese Polymere sind Lösungsmittel-frei (z.B. organische Lösungsmittel wie Ether, Acetonitril, Petrolether oder Wasser), aber ionen-leitfähig (Transport-Eigenschaften besitzen) und können nach Abkühlen auf Raumtemperatur feste Lösungen von dissoziierbaren Elektrolyten (pharmazeutische Wirkstoffe) bilden.

Eine weitere Aufgabe der Erfindung besteht in der Schaffung eines Verfahrens zur Herstellung der erfindungsgemäßen pharmazeutischen Zubereitung in Form eines isotropen Einphasen-Polymer-Systems.

Diese Aufgabe wird erfindungsgemäß durch die Bereitstellung eines Verfahrens gelöst, welches folgende Verfahrensschritte umfaßt :

a.) Erwärmen des Trägers unter Rühren bis oberhalb des Schmelzpunktes, bis eine isotrope, transparente Flüssigkeit vorliegt;

b.) Messung der elektrischen Leitfähigkeit und der Viskosität bei der Temperatur des Schmelzpunktes, um das Vorliegen einer isotropen, transparenten Flüssigkeit zu gewährleisten;

c.) Bestimmung des refraktiven Index;

d.) Einstellen der gewünschten Konzentration des pharmazeutischen Wirkstoffs unter Beachtung des Molenbruches, der bei 37° C zwischen 0,001 und 0,67 liegen muß;

e.) Eintragen des pharmazeutischen Wirkstoffes unter stetigem Rühren in das Lösungsmittel;

f.) Rühren des Ansatzes, bis der pharmazeutische Wirkstoff gelöst und eine transparente Lösung entstanden ist;

g.) Messung des differentiellen refraktiven Index-In crementes [$(\Delta n/\Delta c)_{T/P=konstant}$] zur Bestimmung der monomolekularen Lösung und/oder

h.) Kontrolle der nativen Konformation und der Monomolekularität des pharmazeutischen Wirkstoffes in der Lösung durch Messung des molaren Extinktionskoeffizienten im UV-Bereich und durch Aufnahme des Absorptionsspektrums sowie Feststellen der chiralen Konfiguration durch Messung im Polarimeter und/oder

i.) Messung der Trübung, um eine homogene Lösung sicherzustellen und/oder

k.) Messung der spezifischen Leitfähigkeit $[(\Lambda)_{T,V=konstant}]$ zur Kontrolle der ionalen Konzentration in der isotropen Lösung;

l.) Abkühlen der klaren Lösung und Herstellung einer galenischen Formulierung ;

m.) weiteres Abkühlen der Lösung auf Raumtemperatur, bis die Lösung erstarrt ist.

Die Herstellung der pharmazeutischen Zubereitung erfolgt also z.B. erfindungsgemäß in der Weise, daß die entsprechende Menge Matrix, z.B. Polyethylenglykol 1500 oder Polyethylenoxid, in einem Behälter bei 55 °C geschmolzen wird, wobei der pharmazeutische Wirkstoff unter stetigem Rühren eingetragen wird. Hierbei ist zu beachten, daß immer eine transparente Lösung beim Eintragen des Wirkstoffs bestehen bleibt, so daß weder eine Entmischung noch eine trübe Lösung entsteht. Die oben angeführten Kontrollen bzw. Messungen sind hierbei im einzelnen unbedingt durchzuführen. Es entsteht somit gemäß dieses Verfahrens eine homogene Lösung. Diese klare Schmelze kann auf ungefähr 35 - 40 °C abgekühlt werden und sodann in Hartgelatinekapseln der Größe 0 dosiert werden. Dies kann u.a. mit einer GKF 1500 Einrichtung von Höfliger und Karg, die mit einer entsprechenden Dosiereinheit ausgerüstet ist, durchgeführt werden. Nach dem Abfüllvorgang erstarrt die Schmelze nach Abkühlen auf Raumtemperatur in der Kapsel zu einem festen kristallinen oder nicht-kristallinem Körper, je nach pharmazeutischen Wirkstoff. Die pharmazeutischen Wirkstoffe bestimm ter Konzentrationen werden nach Wägung oder nach Messen des molaren Extinktionskoeffizienten, z. B. bei Proteinen bzw. Peptiden, in geeigneten Lösungsmitteln einschließlich wäßriger oder organischer Lösungsmittel dosiert in die Schmelze eingegeben, indem man die entsprechende Konzentration in Lösung spektralphotometrisch bestimmt hat, oder nach Messung der Extinktion (Konzentration) die wäßrige Lösung (oder organische Lösung) lyophilisiert und das Lyophilisat (fest) wie vorher beschrieben als Feststoff PEG einsetzt.

Ein anderes Verfahren besteht darin, daß man den pharmazeutischen Wirkstoff gleich in Polyoxyethylenglykol oder Polyethylenoxiden unter Beachtung des Molenbruches in einer bestimmten Konzentration der Schmelze bei 50 °C löst, spektralphotometrisch die Konzentration über den Extinktionskoeffizienten bestimmt und anschließend mit der Matrix auf die gewünschte Konzentration bei 50 °C reduziert bzw. einstellt.

Die Erstarrung bei der Kristallisation der Schmelze kann beschleunigt bzw. auch initiiert werden durch Zugabe von kristallinen pharmazeutischen Wirkstoffen, die vor oder nach Abfüllen der Schmelze in die Kapsel eingefüllt werden. Dies ist u.a. wichtig hinsichtlich der Erstarrung von Schmelzen bei chiralen pharmazeutischen Wirkstoffen, z.B. (S)-Ibuprofen, um die Erstarrung zu beschleunigen. Es handelt sich hier um eine Nukleationsreaktion, die die Keimbildung von Kristallen beschleunigt, wie sie bei normalen echten Lösungen beobachtbar ist.

Eine erfindungsgemäße Ausführungsform besteht darin, daß man die Kristallisation bzw. die Erstarrung durch mikrokristalline Zugabe von (R,S)-Ibuprofen initiiert, um feste Lösungen von (S)-Ibuprofen zu erhalten.

Erfindungsgemäß kann auch Polyethylenglykol-660-hydroxystearat (Solutal HS 15) als inerte Matrix eingesetzt werden.

Im folgenden werden die erfindungsgemäß vorzugsweise einsetzbaren Wirkstoffe und ihre Indikationen offenbart:

| | | |
|---|---|---|
| Antikörper | Anti-Rh-Globulin | Vorbeugung von Rh-Isoimmunisierung |
| | Immun-Globuline | Immundefizit |
| | spez. Immun-Globuline | passive Immunisierung Hepatitis B Pertussis Tetanus |
| Antigene | Vakzine | Aktive Immunisierung, Mumps, Hepatitis |
| Enzyme | Asparaginase | Leukämie |
| | Chymopapain | Bandscheibenvorfall, |
| | Collagenase | Topisch |
| | Hyaluronidase | SQ-Absorption |
| | Leukase, Trypsin | Wunden |
| | Plasminogen Aktivatoren | Thrombolysis |
| | Thrombin | Topisch, Hämostasis |
| Hormone | Insulin | Diabetis mellitus |
| | Oxytoxin | Diabetis insipidus |
| | Vasopressin | Diabetis insipidus |
| | menschliches Wachstumshormon | |
| | Gonadotropine | Induzierung der Ovulation |
| | (hCG; LH, FSH etc. | Spermatogenese |

| | | |
|---|---|---|
| Immun. Faktoren | Interferon-α | Haarzellen-Leukämie |
| Verschiedenes | Aspartam | Zucker-Ersatz |
| | Serum-Albumin | Plasma-Expander |
| | Protein-hydrolysate | Ernährung (Kinder) |
| Digoxin | | Herzmuskel |
| | Cyclosporin | Antibiotisch |
| | Collagen | künstliche Transplantate |

Als Träger werden erfindungsgemäß vorzugsweise Polyethylenglykole, vorzugsweise in einem Molekulargewichtsbereich von 400 - 6000, verwendet. Weiterhin werden erfindungsgemäß vorzugsweise Propylenoxide, vorzugsweise mit einem Molekulargewicht von 600 - 6000, eingesetzt.

Erfindungsgemäß können weiterhin vorteilhaft Paraffin-Ester, z.B. Isopropylpalmitat und/oder Isopropylmyristat, eingesetzt werden. Weitere inerte Füllmassen sind Produkte, die häufig als Verdickungsmittel verwendet werden, wie z.B. Stearylalkohol. Ganz besonders bevorzugt wird Polyethylenglykol und Polyoxyethylen oberhalb etwa 1000 und vorzugsweise einem Molekulargewicht von 1500. Voraussetzung für die Eignung der inerten Füllmasse bei der vorliegenden Erfindung ist neben der schon erwähnten Inertheit, daß diese Füllmasse bei Körpertemperatur schmelzflüssig ist oder wird.

Anstatt Polyoxyethylenglykol oder Polyethylenglykol können auch Polypropylenglykol W1,2) oder auch Propylenglykolmonostearate eingesetzt werden, insbesondere bei Alkalisalzen von pharmazeutischen Wirkstoffen, die eine Carboxyl- oder primäre Hydroxylgruppe tragen.

Vorzugsweise wird die erfindungsgemäße pharmazeutische Zubereitung in Hartgelatinekapseln als Hülle eingebracht. Als Hülle kommt erfindungsgemäß weiterhin ein Film in Betracht, der aus dem oben bereits beschriebenen Trägermaterial bestehen kann.

Im folgenden werden Versuchsbeispiele beschrieben, die insbesondere die Struktur und Wirkung sowie wesentliche Vorteile der erfindungsgemäßen pharmazeutischen Zubereitung betreffen:

Physikalisch-chemische Untersuchungen zeigen, daß es sich um ein isotropes Einphasen-Polymer-System sowohl im flüssigen Zustand als auch nach Abkühlen in den festen Zustand bei Raumtemperatur handelt. Dadurch entgeht man allen Schwierigkeiten bezüglich der Diffusion des pharmazeutischen Wirkstoffes oder des Freisetzungs-Verhaltens in vivo und in vitro (bessere Anflutungszeiten bzw. Bioäquivalenz) durch eine nicht-einheitliche Verteilung des Wirkstoffes innerhalb des Polymer-Elektrolyten. Ein weiterer Vorteil besteht in der Vermeidung von verschiedenen, isotropen Phasen, z.B. kristallin-amorph und Phasen-Separierung bzw. Ausbildung a.) nach Auflösen in wäßrigen (physiologischen) Medien, b.) nach Schmelzen dieser pharmazeutischen Formulierung bei 37-40° C, so daß c.) ein isothermaler Transport des pharmazeutischen Wirkstoffes gewährleistet ist.

Die Elektrolyt-Fähigkeit der Matrix als Lösungsmittel für die pharmazeutischen Wirkstoffe kann u.a. dadurch belegt werden, daß $LiClO_4$ mit bzw. ohne Wirkstoff die Leitfähigkeit im geschmolzenen (flüssigen) Zustand bzw. die Diffusionskoeffizienten des Wirkstoffes beeinflussen; die Diffusionskoeffizienten nehmen mit zunehmender Konzentration des Wirkstoffes, bei Zunahme der Träger-Elektrolyten ($LiClO_4$) sowie bei Verringerung der Temperatur ab. Diese Konzentrationsabhängigkeit kann durch die "Freie Volumen-Theorie" quantitativ erklärt werden (M.H. Cohen, D. Turnbull, J. chem. Phys. 1959, 31, 1164). Durch das ausgiebige Rühren der flüssigen Matrix nach Eingabe des pharmazeutischen Wirkstoffes bilden sich im Fall von PEO-Polymer-Elektrolyten solvatisierte Ionen durch dipolare bzw. Kordinations-Wechselwirkungen aus, speziell bei organischen Anionen oder anorganischen Kationen. Diese Wechselwirkungen reduzieren insbesondere die segmentale Mobilität der Polymerkette. Diese Einschränkung der Flexibilität ist natürlich abhängig von der Konzentration des pharmazeutischen Wirkstoffes (Molenbruch). Diese Abnahme der

segmentalen Flexibilität ist außerdem begleitet von einer Abnahme des Diffusionskoeffizienten des pharmazeutischen Wirkstoffes innerhalb der Polymer-Matrix, die Rück-Verteilung des freien Volumens, durch welches die Diffusion des Wirkstoffes gewährleistet wird, welche gleichzeitig mit der segmentalen Polymer-Beweglichkeit von statten geht. Nur für geringe Konzentrationen an pharmazeutischen Wirkstoff (Molenbruch ≈ 0.001) besteht keine Konzentrationsabhängigkeit des Diffusionskoeffizienten mit der Konzentration des Wirkstoffes. Ein weiteres Charakteristikum dieser pharmazeutischen Formulierung besteht darin, daß nach Ausbildung eines Ions des pharmazeutischen Wirkstoffes unter Beibehaltung der stereochemischen Konformation, z.B. durch die Dissoziation in ein Anion und Proton (S-(+)-Ibuprofen-C00H = $H^+$ + S-(+)-Ibuprofen $C00^-$), diese ein typisches Nicht-Arrhenius-Verhalten zeigen, das sehr ähnlich dem entspricht, was man bei Ionen vorfindet, die sehr stark mit der PEO-Matrix als einfache, inerte Moleküle interagieren.

Die erfindungsgemäßen molekularen Lösungen wurden sowohl im erstarrten als auch im geschmolzenen Zustand bei variablen Konzentrationsverhältnissen von inerter Matrix (Träger), z.B. Polyethylenglykol 1500 oder Polyoxyethylenoxid 2000, und pharmazeutischen Wirkstoffen durch Röntgenkleinwinkel (RKWS)- und Neutronen-Streuung gemessen. Da dies keine invasive Methodik ist und außerdem noch thermodynamische Größen bestimmt werden, konnten diese Messungen auch in Lösung bei ca. 37° C nach Freisetzung aus der Hartgelatinekapsel durchgeführt und mit den vorstehend beschriebenen Experimenten verglichen werden. Es wurde überraschend gefunden, daß, wie zunächst am Beispiel S-(+) Ibuprofen, (R,S) Ibuprofen und R-(-) Ibuprofen als Wirkstoffe und Polyethylenglykol 1500 bzw. Polyethylenoxid 1500 als inerte Matrix, gezeigt werden konnte, diese Schmelze (flüssig) mit einer verdünnten Polymerlösung (inerte Matrix) und mit einem gelösten Stoff (pharmazeutischer Wirkstoff) sich gemäß den physikalisch-chemischen Gesetzen verhält. Das gleiche gilt für die erstarrte Schmelze. Die Streuungskurven zeigen amorphes Verhalten ("strukturloses Verhalten") im Sinne einer diffusen Streuung, so daß die wichtigsten thermodynamischen Parameter bestimmt werden konnten. So ist u.a. die Bildung von geordneten Gebilden ("clustering") von S-(+) Ibuprofen sowohl in der Schmelze als auch im erstarrten Zustand an wasserlösliche (hydrophile) lineare Makromoleküle wie PEG 1500 oder PEO 1500 festzustellen. Daher können PEG 1500 und/oder PEO 1500 eine große Anzahl von S-(+) Ibuprofen-Molekülen einbinden. Dies gilt für die protonierten razemischen oder enantiomeren Verbindungen der Profenreihe als auch für die Alkali- oder Tromethamin-Salze. So wurde u.a. gefunden, daß PEG 1500 oder PEO 1500 in der Schmelze (flüssig) durchschnittliche, gewichtsmäßige Molekulargewichte von ca. 25.000 - 30.000 haben, ähnliche Werte wurden für die erstarrte Schmelze von $MW_r$ = 28.500 ± 5.900 durch RWKS bestimmt. Der durchschnittliche, elektronische Massenradius beläuft sich auf ca. Rg = 45.0 ± 5,0 Å, so daß die Konformation der linear verknüpften $-CH_2-$ oder $-OCH_2$-Gruppen einer linearen Zick-Zack-oder Mäanderform entsprechen. Durch Zugabe von S-(+) Ibuprofen der R-Form oder auch des Razemates ändert sich $MW_r$ in Abhängigkeit von der Konzentration des pharmazeutischen Wirkstoffes. Dieser Vorgang kann sehr gut durch Änderung des 2. Virialkoeffizienten oder der isothermen Kompressibilität eines Einkomponentensystems (Lösung) im physikalisch-chemischen Sinne beschrieben werden. So bewirkt z.B. eine Änderung der Zugabe eines pharmazeutischen Wirkstoffs mit niedrigem Molekulargewicht, z.B. die der Profene, Pindolol, Dilthiazem, Mofebutazon, gegenüber größeren und oberflächenaktiven Stoffen, wie z.B. die Prostaglandine ($PGE_2$, $PGF_{2\alpha}$, $PGA_1$ oder $PGB_1$), Leukotriene ($LTB_4$, $LTD_4$) oder Oligopeptide, wie lineares Tyrocidin, Vasopressin, oder das Peptid des natrialen Faktors (NAF), eine Zunahme des ausgeschlossenen Volumens dieser letzteren Arzneistoffe innerhalb der Schmelze und somit eine Abnahme des 2. Virialkoeffizienten. Da die Konzentrationen an Arzneistoffen innerhalb der Schmelze (Lösung) molarmäßig gering ist, z.B. für NAF≈10nM, $PGE_2$≈100µm, $PGF_{2\alpha}$≈10µM, Vasopressin≈10mM (≈20µg), kann man diese Lösungen als verdünnte, homogene Lösungen aus pharmazeutischen Wirkstoffen bezeichnen, die den normalen physikalisch-chemischen Gesetzen gehorchen. Die mikroskopische Erklärung für diese Phänomene bei diesen sehr unterschiedlichen Arzneistoffen liegt in der hydrophoben Bindung von einigen $CH_2$-Gruppen der Polymere in exponierten hydrophoben Regionen der Arzneistoffe, z.B. die hydrophobe Tetraenkette der Prostaglandine, den p-substituierten Bereich des Ibuprofens einschließlich des Phenylringes und des Phenylrestes beim Tyrocidin oder Gramicidin etc. an der Oberfläche dieser Arzneistoffe. Außerdem konnten Komplexmechanismen von $Na^+$, $K^+$, $Li^+$ oder $NH_4^+$ bei PEG oder PEO an Polymer-Loops festgestellt werden. Die freie Energie δG der Bindung von Arzneistoffen der Profenreihe liegt bei 0,5-0,7 $k_B$ .T/PEG Molekül, die der Prostaglandine bei 1,0 - 1,2 $k_B$.T/PEG. Das δG für S-(+) Ibuprofen wurde zu 0,56 $k_B$.T/PEG oder 0,5 $k_B$.T/PEO bestimmt, ähnliche Werte für das R-Enantiomere; die (R,S)-Form hat jedoch einen δG-Betrag von 2,5 $k_B$.T/PEG. Dies liegt an der Dimerisierung der R- und S-Form innerhalb des Razemates, so daß ein zusätzlicher Betrag in Form der Mischungsentropie aufgebracht werden muß. Dies macht sich u.a. auch in der drastischen Änderung des partiellen spezifischen Volumens, $v_2$, bemerkbar, welches bei den Razematen unproportional zunimmt. Dies gilt generell für Razemate gegenüber Enantiomeren bei diesem

System bestehend aus inerter Matrix und Wirkstoff, wobei das Enantiomer thermodynamisch stabiler ist. (Razemate, insbesondere der 2-Aryl-Propionsäuren, können sich unkontrolliert in Enantiomere separieren und anschließend razemische Conglomerate bilden, die aufgrund der verschiedenen Protonenaktivitäten am chiralen Kohlenstoffatom entstehen können.

Die micell-formenden Prostaglandine wie $PGE_2$ (Dinoproton), $PGE_{2\alpha}$ (Dinoprost),
oder deren Tromethamin-Salze bei deprotonierten Verbindungen, die hohe therapeutische Bedeutung haben, bilden stabile Komplexe in der Schmelze mit PEG oder PEO, z.B. durch Ausbildung von Wechselwirkungen der protonierten Carboxylgruppen entweder mit $-OCH_2$-Gruppen (Etherbrücke, COOH - O $-CH_2$-) oder in der deprotonierten Form, $-COO^-$ mit dem Hydroxyl von PEG oder einem H-Atom der PEO-Gruppe. Dies erklärt die drastischen Konformationsänderungen, Mäander-Zick-Zack-Konformationen, welche sich u.a. auch durch FT-IR-Messungen und Bestimmung der optischen Rotationsdispersion sowie in der Änderng des elektronischen Massenradius manifestieren.

Die geometrische Anordnung dieser erfindungsgemäßen Schmelze ist ähnlich einer "Halskette" mit der Maßgabe, daß das PEG oder PEO-Polymer sich um die "micellaren" "Knäuel" der Prostaglandine umschließt, so daß die PEG's entlang des Polymers aufgereiht sind. Diese Situation wird beim In-Lösunggehen in ein wäßriges Medium nach Aufschmelzen der Hartgelatinekapsel durch Anlagerung von Wasser noch verstärkt, so daß nur eine protektive Wirkung der PEO- oder PEG-Polymere besteht. Dies trifft insbesondere für Wirkstoffe wie Pentoxyfyllin, Triamteren und substituiertem Triamteren und deren Salze und Amilorid zu, wobei die protektive Wirkung gegenüber den Zellen der Pentagastrin-Produktion durch diese erfindungsgemäße pharmazeutische Formulierung durch intermolekulare Wechselwirkungen verstärkt wird.

Die Abbildungen 1 und 2 zeigen Streuungskurven für PEG 1500-Schmelzen und PEO 2000-Schmelzen in Gegenwart von S-(+)-Ibuprofen, Vasopressin und linearem Tyrocidin A.

Im Gegensatz zur Thermodynamik von Substanzen wie PEG und PEO in der Schmelze sowie in wäßriger Lösung zeigen die Hauptmaxima der Experimente (Abb. 1 und 2) keine Interpartikel-ferenz-Effekte. Während die wäßrigen Lösungen von PEG und PEO mit steigender Temperatur ein typisches Demixing-Phänomen zeigen, ist dies bei den Schmelzen mit pharmazeutischem Wirkstoff nicht zu beobachten: dies gilt überraschenderweise auch für die wäßrige Lösung bei 37°C - 40°C von PEG und PEO und S(+)-Ibuprofen oder der nicht-cyclischen Form von Mofebutazon (2-[S]-n-Butyl-1-phenyl-hydrazinocarbonyl-hexansäure) nach Freisetzung aus der Hartgelatinekapsel (Abb. 3). Normalerweise liegen die physikalisch-chemischen Verhältnisse bei Mischungen so, daß mit steigender Temperatur das Demixing-Phänomen (Phasenstreuung) einsetzt, wenn je nach Zusammensetzung der Mischung die Phasentemperatur erreicht wird, wobei diese Divergenz begleitet wird von einer Änderung der Kompressibilität und der Vorwärtsstreuung ($S(O) = \rho \, . \, k_B \, . \, T \, . \, x_t$), wobei $x_t$ die osmostische Kompressibilität ist und $\rho$ die Elektronendichte. Da bei diesem Phänomen die abstoßenden Kräfte überwiegen, wird die osmotische Kompressibilität reduziert und somit der Strukturpeak im Hauptmaximum der RKWS-Kurve kleiner, so daß ein zweiter (in kleineren Bragg-Winkeln) Interaktionspeak entsteht. Dies ist gemäß der Erfindung nicht der Fall, da die repulsiven Kräfte durch den Zusatz der pharmazeutischen Wirkstoffe reduziert werden und somit sowohl in der Schmelze als auch in der wäßrigen Lösung nach Auflösen aus der Hartgelatinekapsel beim Temperaturen bis etwa 37°C nicht wirksam werden können.

Als experimentelle Beispiele werden die charakteristischen diffusen Streuungskurven u.a. für S-(+)-Ibuprofen, gelöst in PEG 1500 bei höheren Streuungsvektoren dargestellt (Abb. 4 und 5).

Aus den Experimenten kann man schließen, daß jeder pharmazeutische Wirkstoff sowohl in der Schmelze als auch in der späteren wäßrigen Lösung nach Inlösungsgehen aus der Hartgelatinekapsel bei ca. 37°C mit einem Aggregat von PEG oder PEO umgeben ist. Daher ist der elektronische Massenradius von PEG und/oder PEO vergleichbar mit dem "freien" PEG oder PEO "Coil"- (Konformation), z.B. in diesem Fall für S-(+)-Ibupro fen von 15 Å für M =1.500 oder PEO von 150 Å bei M = 12.000. Die pharmazeutischen Wirkstoffe, insbesondere S-(+)-Ibuprofen und die S-Form der nicht-zyklischen Verbindungen von Mofebutazon sind gleichsam als "Cluster" (Untereinheiten) je nach Ionenstärke in dieses "Coil"-Netz eingebettet. Die absorbierte Strecke an der PEO oder PEG-Matrix durch die pharmazeutischen Wirkstoffe hängt ausschließlich von der Konzentration der Wirkstoffe, Temperatur und Ionenstärke, und Zusammensetzung der wäßrigen Lösung ab.

Daher kann man die erfindungsgemäße pharmazeutische Zubereitung als eine verdünnte Lösung von unabhängigen gelösten Arzneistoffen bezeichnen, die als Lösungsmittel u.a. PEG oder PEO-Einheiten tragen. Es handelt sich also weder um ein Gel noch um eine Mikroemulsion oder Suspension.

Unter fester Lösung wird erfindungsgemäß verstanden, daß ausgesprochene starke Wechselwirkungen zwischen den Molekülen A (Matrix, PEO, PEG) und B (pharmazeutischer Wirkstoff) auftreten, und zwar dann, wenn der Stoff B in einer sehr großen Menge des Lösungsmittels A aufgelöst wird. Die Solvatation

der B-Moleküle bewirkt in diesem Fall, daß die Lösung als eine ideale Mischung von A- und solvatisierten B-Molekülen aufgefaßt werden kann, in der der Zustand der B-Moleküle zwar gegenüber ihrem Zustand in der reinen B-Phase stark verändert, aber doch weitgehend konzentrationsunabhängig ist, solange er der maximalen Solvatation entspricht. Dieser (ideale, verdünnte) Zustand wird je nach den individuellen Eigenschaften des Stoffes B bei größeren oder auch kleineren Molenbrüchen erreicht. Bei Systemen fest/flüssig und auch gasförmig/flüssig ist die flüssige Komponente eindeutig das Lösungsmittel und der Festkörper - hier der gelöste Stoff. Da das Lösungsmittel bei ca. 37° C flüssig ist (Molekül A) und der pharmazeutische Wirkstoff (Molekül B) eindeutig ein fester oder flüssiger Stoff ist, kann in diesen Lösungssystemen der Molenbruch des Lösungsmittels gegen 1 gehen, so daß eine unendliche verdünnte Lösung bei 37° C, unterhalb 37° C als feste Lösung, entsteht.

Es gelten also für die hier beschriebene Lösung das Lösungsgleichgewicht: chemisches Potential des gelösten Stoffes (Moleküle B) im festen Zustand = chemisches Potential dieses Stoffes der Lösung, z.B. bei 37° C in der Matrix (Molekül A) : Daraus ergibt sich, daß der Standardwert des chemischen Potentials der gelösten Stoffe in dieser Lösung (Schmelze ca. 37° C) der Anteil des chemischen Potentials ist, der sich bei Extrapolation auf unendliche Verdünnung als konzentrationsunabhängig erweist.

$$\mu_1^0 (T) = \lim_{c \to 0} (\mu_1 (T,C) - R \cdot T \ln c)$$

Im Gegensatz zu Mischsystemen flüssig/flüssig oder Emulsionen, bei denen die chemischen Potentiale der reinen Komponenten als Standardwerte verwendet werden, wird somit beim chemischen Potential der hier gelösten Stoffe der Zustand unendlicher Verdünnung für den Standardwert herangezogen. Bei höheren Konzentrationen müssen die Abweichungen vom idealen Verhalten gemäß Kompressibilität, Virialkoeffizienten, berücksichtigt werden (z.B. $A^*_{osm} = R \cdot T\ 1n^c/c_{id} + A$; $A \equiv R \cdot T\ \ln fa$)

Verfolgt man die Löslichkeitskurve von z.B. (S)-Ibuprofen in Polyethylenoxid oder Polyethylenglykol 1500 im Bereich unter 30° C, in der man die Lösungen im Sättigungszustand entsprechend abkühlt, so kommt man schließlich zu einer Temperatur, bei der außer dem (S) oder (R)-Ibuprofen auch das Lösungsmittel, hier Polyethylenglykol oder Polyethylenoxid, kristallisiert, d.h. die gesamte Lösung erstarrt (siehe Abb.6, Punkt a). Der feste Zustand stellt darum ein Gemenge von (S)-Ibuprofen (oder (R)-Ibuprofen) und Polyethylenoxd-Kristallen dar und kann als "Kyro-Solvat" bezeichnet werden, ohne daß die Lösung mehrere Phasen bildet, also nicht heterogen ist bzw. wird.

Die Funktion der Matrix als Lösungsmittel und damit verbunden die Erhaltung der stereochemischen Konformation entsprechend der erfindungsgemäßen Herstellung dieser pharmazeutischen Zubereitungen läßt sich sehr eindrucksvoll durch die Bestimmung der absoluten Konformation z.B. als (R) oder (S)-Ibuprofens aus dieser Schmelze durch Röntgenstrukturanalyse beweisen. Die aus der Schmelze isolierten Einkristalle, welche aus einer gesättigten Lösung von (S)- bzw. (R)-Ibuprofen in Polyethylenoxid bzw. Polyethylenglykol 1500 erhalten wurden, haben folgende kristallographische Zelldimensionen : für (R)-Ibuprofen.

a = 12,46(1) Å
b = 8,02(1) Å
c = 13,53(1) Å
$\beta$ = 112,97(9)°
V = 1245, (5) Å$^3$
Raumgruppe R2₁, gemessene Dichte : 1,105 g/cm$^3$, berechnete Dichte für Z = 4
d = 1,100 g.cm$^{-3}$
entsprechende Werte für S-Iboprofen wurden ermittelt zu :
a = 12,437(5) Å
b = 8,0104(2) Å
c = 13,500(4) Å
$\beta$ = 112,9(2)°
V = 1239 Å$^3$

Raumgruppe P2₁ gemessene Dichte der Einkristalle von S-Ibuprofen : 1,105 g/cm$^3$ berechnete Dichte für Z = 4 und F.MW = 206,28 $d_{calc}$ = 1,106 g/cm$^3$ . Einkristalle von (S)- bzw. (R)-Ibuprofen, welche aus ethanolischen Lösungen gezogen worden sind, ergaben die gleichen Zelldimensionen wie die oben angeführten Werte. Sowohl die Kristallgitter von (S)- bzw. (R)-Ibuprofen (Abb. 7 und 8) als auch die Konformation der Enantiomeren (Abb. 8 und 9) sind identisch mit den Kristallen der Enantiomeren, die aus

der erstarrten Schmelze erhalten wurden und mit denen aus alkoholischer Lösung. Wie man den Abbildungen entnehmen kann, besteht die asymmetrische Einheit aus zwei (S)- bzw. (R)-Ibuprofen-Molekülen, die über die Karboxylatgruppe durch Wasserstoffbrückenbindungen miteinander verbunden sind. Sowohl die Bestimmung der absoluten Konformation von (S)- und (R)-Ibuprofen als auch HPCL-Analysen belegen, daß keine Änderung am chiralen Zentrum eintritt und daß die Konformation gewahrt bleibt. Dies läßt sich u.a. auch für die nicht-zyklische Form von Mofebutazon, Ketoprofen und Naproxen zeigen. Überraschenderweise wurde gefunden, daß der Zusatz von Razemat-Ibuprofen (R,S) zur Beschleunigung der Erstarrung durch Keimbildung nur die entsprechende (R)- bzw. (S)-Form des Ibuprofens oder eines anderen Enantiomeren induziert und nicht die (S)- bzw. (R)-Form des Ibuprofens z.B. razemisiert. Dies ist ein sehr großer Vorteil dieser beschriebenen pharmazeutischen Zubereitung, da z.B. (S)-Ibuprofen bei Anwendung hoher Drucke z.T. in die (R)-Form übergeht (ca. 10 %, Kinetik), oder - wie bei der Röntgenstrukturanalyse gefunden wurde - durch höhere Temperaturen (ca. 25° C - 27° C) und Drücke, wie sie beim Pressen von Tabletten zum Teil angewendet werden (Änderung des molaren Volumens von S-ibuprofen mit dem Druck) und möglicherweise energiereicher Strahlung eine Razemisierung eintritt. Dies tritt bei der hier beschriebenen Schmelze nicht ein. Ähnliche Phänomene wurden auch bei Verwendung von Solutol HS 15 als Lösungsmittel beobachtet.

Die in vitro-Freisetzung des Wirkstoffes, z.B. (S)-(+)-Ibuprofen, aus einer erfindungsgemäßen Kapsel erfolgt wesentlich schneller als aus einer entsprechenden Kapsel, die die gleiche Menge Wirkstoff, jedoch in kristalliner Form, enthält.

Dadurch, daß der Wirkstoff in der Schmelze, die bei Körpertemperatur flüssig ist, enthalten ist, erfolgt eine rasche Resorption und damit ein rascher Wirkungseintritt.

Die in vitro-Freisetzung des Wirkstoffs (S)-(+)-Ibuprofen aus der oben erwähnten Kapsel (Abb. 10) zeigt die deutliche Überlegenheit gegenüber einer Kapsel, die die gleiche Menge Wirkstoff enthält, jedoch in kristalliner Form (Abb.11).

Dies wird eindrucksvoll in vivo durch die Plasmaspiegel, die kürzeren Zeiten von $t_{max}$ und die erhöhte Konzentration von $t_{max}$ im Verhältnis zur Tablette belegt (Abb. 12, 13). Dies wiederum ermöglicht u.a. eine schnellere Beseitigung der Symptome, z.B. Schmerz, Bewegungseinschränkung, als bei einer Tablette, einschließlich einer besseren Bioverfügbarkeit. Diese pharmazeutische Formulierung der Schmelzkapsel hat entsprechend ihrem schnellen Wirkungseintritt eine ähnliche Wirkung wie eine Injektion.

| | |
|---|---|
| $t_{max}$ (Schmelzkapsel) : 25 min<br>$t_{max}$ (Schmelzkapsel) : 28 µg/ml | bei 200 mg<br>S-(+)-Ibuprofen<br>p.o. |
| $t_{max}$ (Tablette) : 119 min<br>$t_{max}$ (Tablette) : 17,5 µg/ml | bei 300 mg<br>S-(+)-Ibuprofen<br>p.o. |

Die kristalline Reinsubstanz (S)-(+)-Ibuprofen läßt sich aber nicht ohne Hilfsstoffe und ohne aufwendige Bearbeitung dosieren. Eine Abhilfe dieser Probleme wäre das Dosieren der Reinsubstanz als Schmelze (52° C). Die in vitro-Freisetzung des Wirkstoffs dieser Hartgelatinekapsel zeigt jedoch einen verzögerten Freisetzungsverlauf (Retardierung) (Abb. 12).

Wie wichtig es für die Freisetzung ist, daß der Kapselinhalt bei Körpertemperatur flüssig ist, zeigt sich deutlich im Vergleich der Freisetzungskurven von zwei gleich gefüllten Hartgelatinekapseln, wovon eine jedoch vor Testbeginn durch Erwärmung aufgeschmolzen wurde (Abb. 13).

Außerdem wurde erfindungsgemäß gefunden, daß nach Herstellung eines dünnen Films, bestehend aus z.B. PEG 1500 oder PEO 2000, nach Abkühlung einer Schmelze, bestehend aus eben dieser inerten Matrix und einem pharmazeutischen Wirkstoff, nicht nur transdermale Systeme aufgebaut werden können, sondern auch, z.B. für Prostaglandine (Dinoprost), kontrollierte, einschließlich pH-kontrollierte Freisetzungen an pharmazeutischen Wirkstoffen erreicht werden können. So sind z.B. $PGE_2$ oder $PGF_{2\alpha}$ als Monomer in wäßriger Lösung aufgrund ihrer oberflächenaktiven Wirkung nicht beständig, wohl aber in Lösung im Film von PEG oder PEO. Andererseits liegt Misoprostol, ein (±) Methyl (11α, 13E)-11,16-dihydroxy-16-methyl-9-oxoprost-13-en-1-oat (diastereamere Mischung), als gelbe, viskose Flüssigkeit vor, welche für therapeutische Zwecke z.B. durch Hydroxypropylmethylzellulose oder Natriumglykolat, mikrokristalliner Zellulose für orale Applikation stabilisiert werden muß. Es kann z.B. sehr leicht in einer therapeutischen Dosis von 200 -400 µM in der hier beschriebenen PEG oder PEO-Schmelze als monomolekulare Lösung stabilisiert

werden und bei ca. 37° C durch Aufschmelzen freigesetzt werden. Auch als Filme können solche pharmazeutische Formulierungen sehr leicht angewendet werden. Sowohl in der Hartgelatinekapsel als auch im Film liegt Misoprostol als Monomer gelöst vor.

Die Funktionstüchtigkeit eines so gearteten Films ließ sich durch cyclische Voltametrie nach Inkorporation eines elektronen-übertragenden Proteins, Cytochrom c, demonstrieren. Wie die Abb. 14 zeigt, ist das cyclische Voltamogramm von Cytochrom c(A) identisch mit dem im Film (B), wenn man Wasser zusetzt.

Dieses Ergebnis steht in Einklang mit den experimentellen Befunden, daß das Proteinmolekül - oder der pharmazeutische Arzneistoff - umgeben ist von einem linearen, flexiblen, ungeladenen, hydrophilen und wasserlöslichen Polymer. Die Diffusion des Proteins oder des Arzneistoffes aus dieser Schmelze bei 37° C ist kleiner, wenn es in Kontakt mit Wasser kommt, z.B. für Cytochrom c bei diesem Film von 2,25 x $10^{-7}$ $cm^2$ . $sec^{-1}$ im Gegensatz zu einer gepufferten, wäßrigen Lösung von 0,95 x $10^{-5}$ $cm^2$ . $sec^{-1}$. Die voltametrischen Untersuchungen können z.B. als Basis für quantitative Untersuchungen von Arzneistoff- sowie Elektronen-Transport in halb-flexiblen Polymeren wie PEG oder PEO dienen.

Erfindungsgemäß handelt es sich um Lösungen, die durch ihre besondere Temperaturabhängigkeit des Diffusionskoeffizienten sowie ihrer spezifischen Leitfähigkeit ein Nicht-Arrhenius- Verhalten zeigen, also nicht linear sind. Dies ist ein Spezifikum dieser Erfindung: eine normale Lösung, wobei das Lösungsmittel ein Polymer ist und wobei nach Auflösen des gelösten Wirkstoffs ein ionaler Aktivitätskoeffizient zusätzlich zum Lösungsmittel sowie alle anderen Größen, wie z.B. der osmotische Druck, Gefrierpunktserniedrigung, usw. gemessen werden können.

Es ist für den Durchschnittsfachmann ohne weiteres ersichtlich, wie er die spezifischen Meßmethoden einzusetzen und durchzuführen hat. Das gilt auch für die erfindungsgemäß verwendeten "polymeren" Lösungsmittel. Die erforderlichen Apparate sind im Handel erhältlich.

Erfindungsgemäß überraschend war, daß das Polymer (z.B. PEG) überhaupt eine "ionale Leitfähigkeit" aufweist, welche nach Auflösen des S-Ibuprofen, z.B. welches ja in wäßriger und etherischer Lösung ($R_1R_2O$ mit $R_1 = R_2 =$ H oder $R_1 = R_2 = C_2H_5$) unlöslich ist, aber in PEO oder PEG ($R_1R_2$-O-$CH_2$-$R_1R_2$-OCH $(OH)_2$) vollständig löslich ist, eine zusätzliche "ionale Leitfähigkeit" bewirkt. Diese Leitfähigkeit nimmt aber mit der Temperatur nicht linear zu, (Arrhenius-Verhalten), sondern hyperbolisch ab (gekrümmt-Nicht-Arrhenius-Verhalten).

Zur Demonstration dieser Verhältnisse kann z.B. die Temperatur-Abhängigkeit des Diffusions-Koeffizienten des gelösten Wirkstoffs (z.B. S-ibuprofen) in PEG bei verschiedenen Molenbrüchen PEG/S-IBP (Konzentrationen von S-IBP/PEO) sowie die ionale Leitfähigkeit exemplarisch dargestellt werden (Fig. 14).

Weitere Aufgabenstellungen, Merkmale und Vorteile der vorliegenden Erfindung ergeben sich aus der folgenden Beschreibung anhand von Ausführungsbeispielen:

## Beispiel 1

500 g Polyethylenglykol 1500 werden in einem Becherglas auf dem Wasserbad bei einer Temperatur von 55° +/- 3° C geschmolzen und gerührt, bis eine isotrope (transparente) Flüssigkeit erhalten wird. Diese isotrope Flüssigkeit wird zur Messung der elektrischen Leitfähigkeit sowie der Viskosität (Rotationsviskosimeter) konstant auf 55 +/- 3° C gehalten, um zu gewährleisten, daß diese Flüssigkeit den Erfordernissen eines Polymer-Elektrolyten entspricht. Zur späteren Bestimmung der klaren Lösung mit dem gelösten S-(+)-Ibuprofen ist es erforderlich, den refraktiven Index mit einem Refraktometer bei (55 +/- 3° C) zu bestimmen. Danach werden 500 g S-(+)-Ibuprofen unter stetem Rühren in die Flüssigkeit eingetragen. Es ist darauf zu achten, daß die Lösung transparent bleibt. Die monomolekulare Lösung von 500 g S-(+)-Ibuprofen in 500 g Polyethylenglykol 1500 wird durch Messung des differentiellen refraktiven Index-Incrementes $(\Delta n/\Delta c)_{T,P = konstant}$, ermittelt. Durch Trübungsmessungen ($\tau$ 600nm) wird u.a. gewährleistet, daß eine homogene Lösung von Gelöstem im Lösungsmittel vorliegt, und keine partikuläre Verteilung von Gelöstem in dem Lösungsmittel. Durch On-line Messung der spezifischen Leitfähigkeit $(\Lambda)_{T,V = konstant}$, wird außerdem die "ionale Konzentration" von S-(+)-Ibuprofen im isotropen Medium kontrolliert.

Zur schnellen und sicheren Erstarrung dieser mono-molekularen Lösung von 500 g S-(+)-Ibuprofen in 500 g Polyethylenglykol 1500 in einer Hartgelatinekapsel ist es erforderlich, mindestens 0,1 % (g/g) kristallines Razemat hinzuzugeben. Diese klare Lösung wird nunmehr auf 40° C +/- 1° C abgekühlt. Mit einer geeigneten Dossiereinheit wird pro Kapsel 600 mg mit dem Unterteil der Kapsel eingeführt. Die Kapsel wird mit dem Oberteil verschlossen und nach dem Ausstoßen aus der Maschine noch auf Raumtemperatur abgekühlt. Die Hartgelatinekapsel braucht nicht mehr versiegelt zu werden. Die Hartgelatinekapsel kann verpackt und gelagert werden.

600 mg Kapselinhalt enthalten :

| | |
|---|---|
| Polyethylenglykol 1500 | 300 mg |
| S-(+)-Ibuprofen | 300 mg |

oder
600 mg Kapselinhalt enthalten :

| | |
|---|---|
| Polyethylenglykol 1500 | 480 mg |
| S-(+)-Ibuprofen | 120 mg |

Beispiel 2

500 g Polyethylenoxid und 500 g S-(+)-Ibuprofen werden, wie in Beispiel 1 beschrieben, bei 45° C ±3° C zu einer klaren Schmelze auf dem Wasserbad unter stetem Rühren erwärmt. Nach dem Abkühlen auf 40° C werden die Hartgelatinekapseln der Größe 0 mit 600 mg Schmelze wie im Beispiel 1 beschrieben, befüllt.

600 mg Kapselinhalt enthalten :

| | |
|---|---|
| Polyethylenoxid | 300 mg |
| S-(+)-Ibuprofen | 300 mg |

Beispiel 3

100 g Polyethylenglykol 200 und 400 g Polyethylenglykol 1000 werden, wie in Beispiel 1 beschrieben, zusammen mit einem Becherglas auf 60° C erwärmt. Es entsteht eine klare Schmelze, der 5 g Pindolol zugegeben wird. Pindolol löst sich bei 60° C in der PEG-Schmelze auf. Nach Abkühlen auf 40° C werden die Hartgelatinekapseln der Größe 0 mit 505 mg der Schmelze in der in Beispiel 1 beschriebenen Weise befüllt :

505 mg Kapselinhalt enthalten :

| | |
|---|---|
| Polyethylenglykol 200 | 100 mg |
| Polyethylenglykol 1000 | 400 mg |
| (R,S)-Pindolol | 5 mg |

Beispiel 4

50 g Diltiazem HCl werden, wie in Beispiel 1 beschrieben, in einer auf 70° C erwärmte PEG-Schmelze, bestehend aus 100 g Polyethylenglykol 200 und 400 g Polyethylenglykol 1000 eingerührt. Das Diltiazem HCl löst sich bei 70° C in dieser Schmelze auf. Die Schmelze wird auf 40° C abgekühlt. Danach werden 550 mg in die Hartgelatinekapseln in beschriebener Weise gefüllt .

550 mg Kapselinhalt enthalten :

| | |
|---|---|
| Polyethylenglykol 200 | 100 mg |
| Polyethylenglykol 1000 | 400 mg |
| Diltiazem HCl | 5 mg |

Beispiel 5

384 g Polyethylenglykol 1000 und 96 g Polyethylenglykol 200 werden, wie in Beispiel 1 beschrieben, auf 44° C erwärmt. 50 mg Arg-Vasopressin werden in die Schmelze eingebracht. Die klare Schmelze wird auf 40° C abgekühlt. 480,05 mg entsprechend 50 µg Arg-Vasopressin/Kapsel werden wie oben beschrieben abgefüllt, Kapselgröße 0. Der Wirkstoffanteil in der Schmelze kann bis auf 100 mg erhöht werden, so daß eine Dosis von 50 µg bis 100 µg resultiert. Die Füllung kann, wie im Beispiel 1 beschrieben, durchgeführt werden.

480 mg Kapselinhalt enthalten :

| | |
|---|---|
| Polyethylenglykol 1000 | 384 mg |
| Polyethylenglykol 200 | 95,05 mg |
| Arg-Vasopressin | 50 µg |

Beispiel 6

50 mg Arg-Vasopressin werden in analoger Weise wie in Beispiel 1 beschrieben mit Polyethylenoxid 2000 (200 g), geschmolzen. Schmelztemperatur 45° C ± 3° C. Die Dosierung beträgt 200,05 mg Schmelze pro Kapsel (Kapselgröße 2) enthaltend 50 µg Arg-Vasopressin.

200 mg Schmelze enthalten :

| | |
|---|---|
| Polyethylenoxid 2000 | 200 mg |
| Arg-Vasopressin | 50 µg |

Beispiel 7

300 g Polyethylenglykol 1000 werden, wie in Beispiel 1 beschrieben, bei 75° C mit 50 g Ketoprofen im Becherglas erwärmt. Es wird eine klare Schmelze erhalten, die durch Rühren homogen gehalten wird. Die Schmelze wird auf 40° C abgekühlt. 350 mg Schmelze, entsprechend 50 mg Ketoprofen/Kapsel, werden in Hartgelatinekapseln der Größe 1 dosiert.

350 mg Schmelze enthalten :

| | |
|---|---|
| Polyethylenglykol 1000 | 300 mg |
| Ketoprofen | 50 mg |

Beispiel 8

200 g Polyethylenoxid 2000 werden auf 45° C ± 3° C erwärmt, wie in Beispiel 1 beschrieben. 400 mg Dinoprost werden der Schmelze zugegeben. Unter Rühren entsteht eine klare Schmelze. Nach dem Abkühlen auf 40° C werden Hartgelatinekapseln der Größe 2 mit 200,4 mg Schmelze gefüllt. Jede Kapsel

enthält 400 µg Dinoprost. Die Abfüllung geschieht in der Art, wie in Beispiel 1 beschrieben.

200,4 mg Schmelze in der Kapsel enthalten :

| | |
|---|---|
| Polyethylenoxid 2000 | 200 mg |
| Dinoprost | 400 µg |

Beispiel 9

In 300 g Polyethylenglykol 1500, welches auf 48° C erwärmt wurde, werden, wie in Beispiel 1 beschrieben, 50 mg Tyrocidin B eingerührt. Es entsteht eine klare Schmelze.

Nach Abkühlen auf 42° C werden Hartgelatinekapseln der Größe 1 mit 300,05 mg Schmelze, entsprechend 50 µg Tyrocidin B/Kapsel gefüllt. Die Abfüllung geschieht in der in Beispiel 1 erwähnten Weise.

An Stelle von Tyrocidin B kann auch Tyrocidin A-D eingesetzt werden. Dabei sind die Einsatzmegen so zu variieren, daß 5 mg Tyrocidn A-D-/Kapsel dosiert werden.

300,05 Schmelze in der Kapsel enthalten :

| | |
|---|---|
| Polyethylenglykol 1500 | 300 mg |
| Tyrocidin B | 50 µg |

Die Erfindung weist insbesondere folgende Vorteile auf

- hohe Dosierungsgenauigkeit
- neutraler Geschmack,
- wenige Hilfsstoffe (nur einer!)
- Verarbeitung des Wirkstoffs ohne vorherige Behandlung wie mahlen, sieben oder granulieren,
- Schutz vor negativen Einflüssen von außen
- kann im Gegensatz zu Weichgelatinekapseln im eigenen Herstellungsbetrieb gefertigt werden,
- als Schmelze kleineres Darreichungsvolumen im Vergleich zu einer Tablette,
- hohe Bioverfügbarkeit;
- schnelle Anflutung, d.h. Erreichen von hohen Plasmaspiegeln ($C_{max}$) bei kürzeren $t_{max}$-Zeiten;
- Vermeidung einer Visko-Elastizität nach Auflösung in wäßrigen Medien, dadurch das Ermöglichen einer normalen Newton'schen Flüssigkeit mit einer wäßrig-ähnlichen Viskosität;
- Erreichen einer hohen Benetzung durch Protektion der hydrophoben Gruppe des pharmazeutischen Wirkstoffs bei allen physiologischen pH's (pH 1,0 - pH 8,0)
- Proteine, Peptide werden normalerweise schnell metabolisiert, insbesondere durch den Angriff von proteolytischen Enzymen im Gastrointestinaltrakt. Hinzu kommen spätere "first-pass-Effekte" durch Leber und Galle, so daß eine orale Therapie nicht von Nutzen ist. Man ist dann auf eine parentale Form angewiesen. Da die meisten kürzeren Peptide, z.B. NAF, durch Prostaglandine und Leukotriene, insbesondere die Peptido-Leukotriene, sehr kurzzeitig in ihrer aktiven Form (unabhängig von ihren Plasma-Halbwertzeiten oder Eliminations-Halbwertzeiten) wirken, müssen gewöhnlich zahlreiche Injektionen verabreicht werden. Durch die Lösung dieser natürlichen, pharmazeutischen Wirkstoffe in PEO, PPO, PEG-Filmen, wie auch bei transdermalen Trägern, können kontrollierte Freisetzungen, einschließlich einer transdermalen ionophoretischen Freisetzung erzielt werden. Diese Stoffe können dosiert am Patienten ohne die oben genannten Nachteile verabreicht werden.

## Ansprüche

1. Pharmazeutische Zubereitung, bestehend aus einem oder mehreren Wirkstoffen und einem Träger, der im Bereich zwischen 20° und 80° C erstarrt und wasserlöslich ist,

**dadurch gekennzeichnet,**

daß sie eine isotrope Lösung ist, wobei a) der Wirkstoff in dem Träger monomolekular oder als Ion gelöst ist,

b) der Wirkstoff in seiner nativen Konformation und/oder seiner biologisch aktiven chiralen (enantiomeren) Konformation vorliegt,

c) der Wirkstoff einen Molenbruch von 0,001 bis 0,67 bei 37° C aufweist,

d) der Träger bei Körpertemperatur schmelzflüssig, phaseneinheitlich und isotrop ist,

e) die isotrope Lösung, bestehend aus Träger und Wirkstoff, bei Raumtemperatur erstarrt,

f) die erstarrte Lösung kristallin oder nicht-kristallin ist oder sie den Wirkstoff kristallin enthält oder sie den Wirkstoff auskristallisieren kann,

g) die monomolekulare oder ionische Lösung einen osmotischen Druck hat und eine molare Gefrierpunkterniedrigung bewirkt,

h) der gelöste Wirkstoff innerhalb des Polymerelektrolyten einen temperaturabhängigen Diffusionskoeffizienten hat und eine temperaturabhängige spezifische Leitfähigkeit hat.

2. Pharmazeutische Zubereitung nach Anspruch 1,
dadurch gekennzeichnet,
daß sie besteht aus 50 - 99,9990 Gew.% Träger und 0,0010 - 50 Gew.% Wirkstoff oder Wirkstoffgemisch, bevorzugt
aus 50 - 55 Gew.% Träger und 45 - 50 Gew.% Wirkstoff oder Wirkstoffgemisch und weiterhin bevorzugt
aus 60 - 85 Gew.% Träger und 15 - 40 Gew.% Wirkstoff oder Wirkstoffgemisch oder
aus 70 - 80 Gew.% Träger und 20 - 30 Gew.% Wirkstoff oder Wirkstoffgemisch oder
aus 75 - 85 Gew.% Träger und 15 - 25 Gew.% Wirkstoff oder Wirkstoffgemisch oder
aus 90 - 99,99 Gew.% Träger und 0,01 - 10 Gew.% Wirkstoff oder Wirkstoffgemisch, wobei sie insbesondere bevorzugt besteht aus 50 Gew.% Träger und 50 Gew.% S-Ibuprofen oder aus 99,98 Gew.% Träger und 0,02 Gew.% Dinoprost oder aus 99,9975 Gew.% Träger und 0,0025 Gew.% Arg-Vasopressin.

3. Pharmazeutische Zubereitung nach einem oder mehreren der vorhergehenden Ansprüche,
dadurch gekennzeichnet,
daß der Träger wasserfrei ist oder
daß die pharmazeutische Zubereitung zusätzlich bis zu ca. 1 Gew.% Wasser oder Ethanol , bezogen auf die gesamte pharmazeutische Zubereitung, enthält, wobei bevorzugt die erstarrte Lösung kristallin ist.

4. Pharmazeutische Zubereitung nach einem oder mehreren der vorhergehenden Ansprüche,
dadurch gekennzeichnet,
daß der Träger ein Polymer mit einem Molekulargewicht von 200 - 10.000 ist, wobei bevorzugt
der Träger ein Polyethylenglykol und/oder
ein Polyoxyethylenglykol und/oder
ein Polypropylenglykol und/oder
ein Polyoxyethylenoxid und/oder
ein Polypropylenoxid und/oder
ein Paraffinester ist, weiterhin bevorzugt,
daß der Träger ein Isopropylpalmitat und/oder ein Isopropylmyristat ist, und
daß der Träger ein Myristylalkohol und/oder ein Palmitinalkohol und/oder ein Stearylalkohol ist.

5. Pharmazeutische Zubereitung nach einem oder mehreren der vorhergehenden Ansprüche,
dadurch gekennzeichnet,
daß der Wirkstoff ein Antikörper ist, wobei bevorzugt
der Wirkstoff Anti-Rh-Globulin und/oder ein Immun-Globulin ist oder,
daß der Wirkstoff ein Antigen ist oder,
daß der Wirkstoff eine Vakzine ist oder,
daß der Wirkstoff ein Enzym ist, wobei bevorzugt
der Wirkstoff Asparaginase und/oder Chymopapain und/oder Collagenase und/oder Hyaluronidase und/oder Leukase und/oder Trypsin und/oder ein Plasminogen-Aktivator und/oder Thrombin ist oder,
daß der Wirkstoff ein Hormon ist, wobei bevorzugt
der Wirkstoff Insulin und/oder Oxytoxin und/oder Vasopressin und/oder ein menschliches Wachstumshormon und/ oder Gonadotropin und/oder hCG und/oder ein lutinisierendes Hormon (LH) und/oder ein Follikelstimulierendes Hormon (FSH) ist oder,
daß der Wirkstoff ein immunogener Faktor ist, wobei bevorzugt
der Wirkstoff α-Interferon ist oder,
daß der Wirkstoff ein Zucker ist, wobei bevorzugt
der Wirkstoff Aspartam ist oder,
daß der Wirkstoff ein Serum-Albumin ist oder,
daß der Wirkstoff ein Protein-hydrolysat ist oder,
daß der Wirkstoff eine antibiotisch wirksame Substanz ist, wobei bevorzugt
der Wirkstoff ein Cyclosporin und/oder cyclisches und/oder lineares Tyrocidin und/oder cylisches und/oder lineares Gramicidin und/oder Thyrotrozin ist oder,

daß der Wirkstoff ein Collagen ist oder,
daß der Wirkstoff ein Anti-Rheumatikum ist, wobei bevorzugt
der Wirkstoff ein nicht steroidales und/oder ein chirales, nicht steroidales Antirheumatikum ist oder,
daß der Wirkstoff ein cardiovaskulärer Wirkstoff ist, wobei bevorzugt
der Wirkstoff Digoxin und/oder ein Prostaglandin und/oder $PGE_2$ ist oder insbesondere bevorzugt,
daß der Wirkstoff S-Ibuprofen ist oder,
daß der Wirkstoff Dinoprost ist oder,
daß der Wirkstoff Arg-Vasopressin ist.

6. Pharmazeutische Zubereitung nach einem oder mehreren der vorhergehenden Ansprüche,
dadurch gekennzeichnet,
daß die pharmazeutische Zubereitung ein isotropes Einphasen-Polymer-System sowohl im flüssigen Zustand als auch nach dem Abkühlen im festen Zustand bei Raumtemperatur ist, weiterhin bevorzugt,
daß der Wirkstoff einen Molenbruch von 0,05-0,25 aufweist, weiterhin bevorzugt,
daß der Wirkstoff im Träger als Anion oder Kation gelöst ist, bevorzugt,
daß die nichtkristalline Lösung auch in erstarrten Zustand eine amorphe Lösung ist, bevorzugt,
daß die erstarrte Lösung mit Wirkstoff kristallin ist.

7. Verfahren zur Herstellung der pharmazeutischen Zubereitung, insbesondere nach einem oder mehreren der Ansprüche 1-6,
gekennzeichnet durch die folgenden Verfahrensschritte:

a.) Erwärmen des Trägers unter Rühren bis oberhalb des Schmelzpunktes, bis eine isotrope, transparente Flüssigkeit vorliegt;

b.) Messung der elektrischen Leitfähigkeit und der Viskosität bei der Temperatur des Schmelzpunktes, um das Vorliegen einer isotropen, transparenten Flüssigkeit zu gewährleisten;

c.) Bestimmung des refraktiven Index;

d.) Einstellen der gewünschten Konzentration des pharmazeutischen Wirkstoffs unter Beachtung des Molenbruches, der bei 37° C zwischen 0,001 und 0,67 liegen muß ;

e.) Eintragen des pharmazeutischen Wirkstoffes unter stetigem Rühren in das Lösungsmittel;

f.) Rühren des Ansatzes, bis der pharmazeutische Wirkstoff gelöst und eine transparente Lösung entstanden ist;

g.) Messung des differentiellen refraktiven Index- Incrementes $[(\Delta n/\Delta c)_{T/P=konstant}]$ zur Bestimmung der monomolekularen Lösung und/oder

h.) Kontrolle der nativen Konformation und der Monomolekularität des pharmazeutischen Wirkstoffes in der Lösung durch Messung des molaren Extinktionskoeffizienten im UV-Bereich und durch Aufnahme des Absorptionsspektrums sowie Feststellen der chiralen Konfiguration durch Messung im Polarimeter und/oder

i.) Messung der Trübung, um eine homogene Lösung sicherzustellen und/oder

k.) Messung der spezifischen Leitfähigkeit $[(\Lambda)_{T,V=konstant}]$ zur Kontrolle der ionalen Konzentration in der isotropen Lösung;

l.) Abkühlen der klaren Lösung und Herstellung einer galenischen Formulierung ;

m.) weiteres Abkühlen der Lösung auf Raumtemperatur, bis die Lösung erstarrt ist.

8. Verfahren nach Anspruch 7,
dadurch gekennzeichnet,
daß nach dem Abkühlen der klaren Lösung die pharmazeutische Zubereitung in Hartgelatinekapseln abgefüllt oder die klare Lösung in Form eines Films abgekühlt wird,
wobei bevorzugt
die klare Lösung auf ca. 35 ° C - 41 ° C abgekühlt und anschließend in Hartgelatinekapseln abgefüllt wird.

9. Verfahren nach einem oder mehreren der vorhergehenden Ansprüche,
dadurch gekennzeichnet,
daß zur schnellen Kristallisation bzw. Erstarrung ein Keimbildner zugegeben wird, wobei bevorzugt
die Lösung mit einem chiralen Wirkstoff auf Zusatz des razemischen Wirkstoffes kristallin oder amorph erstarrt, ohne daß der chirale Wirkstoff razemisiert oder seine Stereochemie verändert, weiterhin bevorzugt,
die Kristallisation bzw. Erstarrung durch mikrokristalline Zugabe von (R,S)-Ibuprofen initiiert wird, um feste Lösungen von (S)-Ibuprofen zu erhalten, wobei bevorzugt der razemische Wirkstoff in einer Konzentration von 0,5 -5 Gew.-%, bezogen auf den chiralen Wirkstoff, zugesetzt wird oder,
daß der razemische Wirkstoff in einer Konzentration von 1 Gew.-%, bezogen auf den chiralen Wirkstoff, zugesetzt wird.

10. Verfahren nach einem oder mehreren der vorhergehenden Ansprüche,
dadurch gekennzeichnet,

daß der Wirkstoff unter Beachtung des Molenbruches zusammen mit dem Träger bis oberhalb des Schmelzpunktes des Trägers erhitzt wird, spektralphotometrisch die Konzentration über den Extinktionskoeffizienten bestimmt wird und anschließend mit dem Träger auf die gewünschte Konzentration eingestellt oder reduziert wird.

Abb. 1

Streuintensität (willkürl. Einh.)

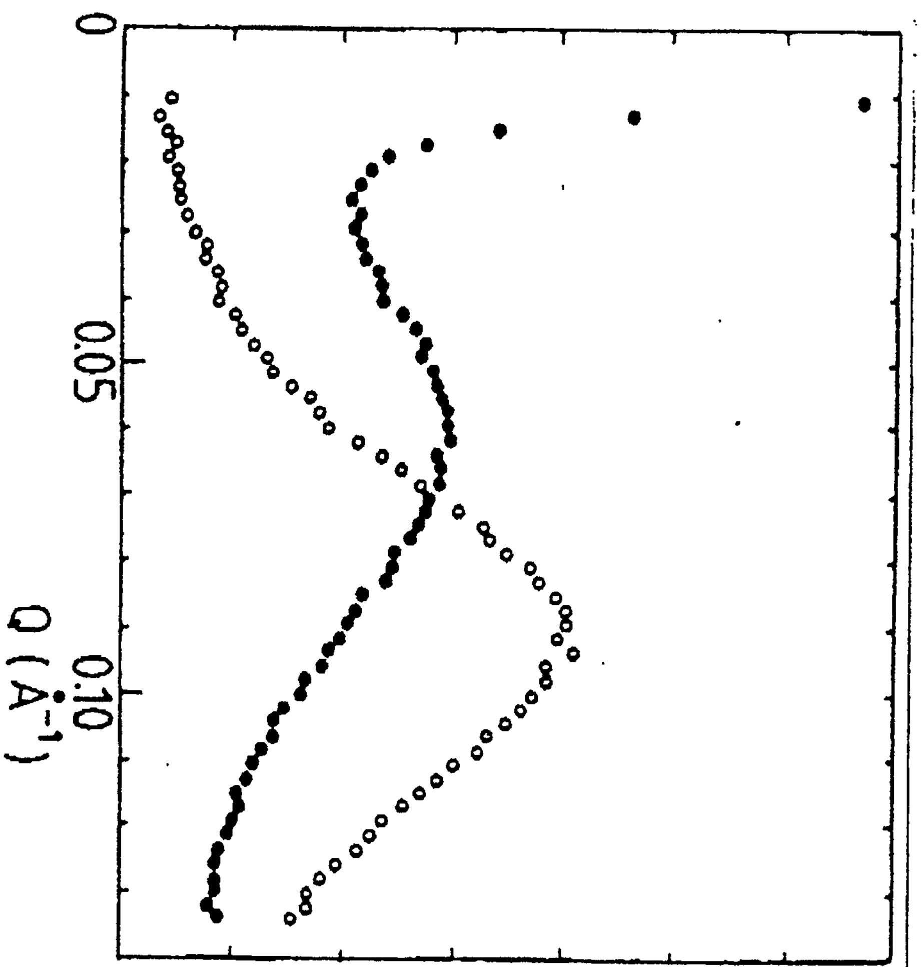


$Q = \frac{4u}{\lambda} \cdot \sin\Theta$, $\lambda$ = Wellenlänge und $\Theta$ = Streuungswinkel

Streuungskurven für PEG 1.500 und S-(+)-Ibuprofen Schmelzen:

o-------o PEG, ●-----● PEO 2000, gemäß Beispiele 1 und 2

Abb. 2

Log (Streuintensität)

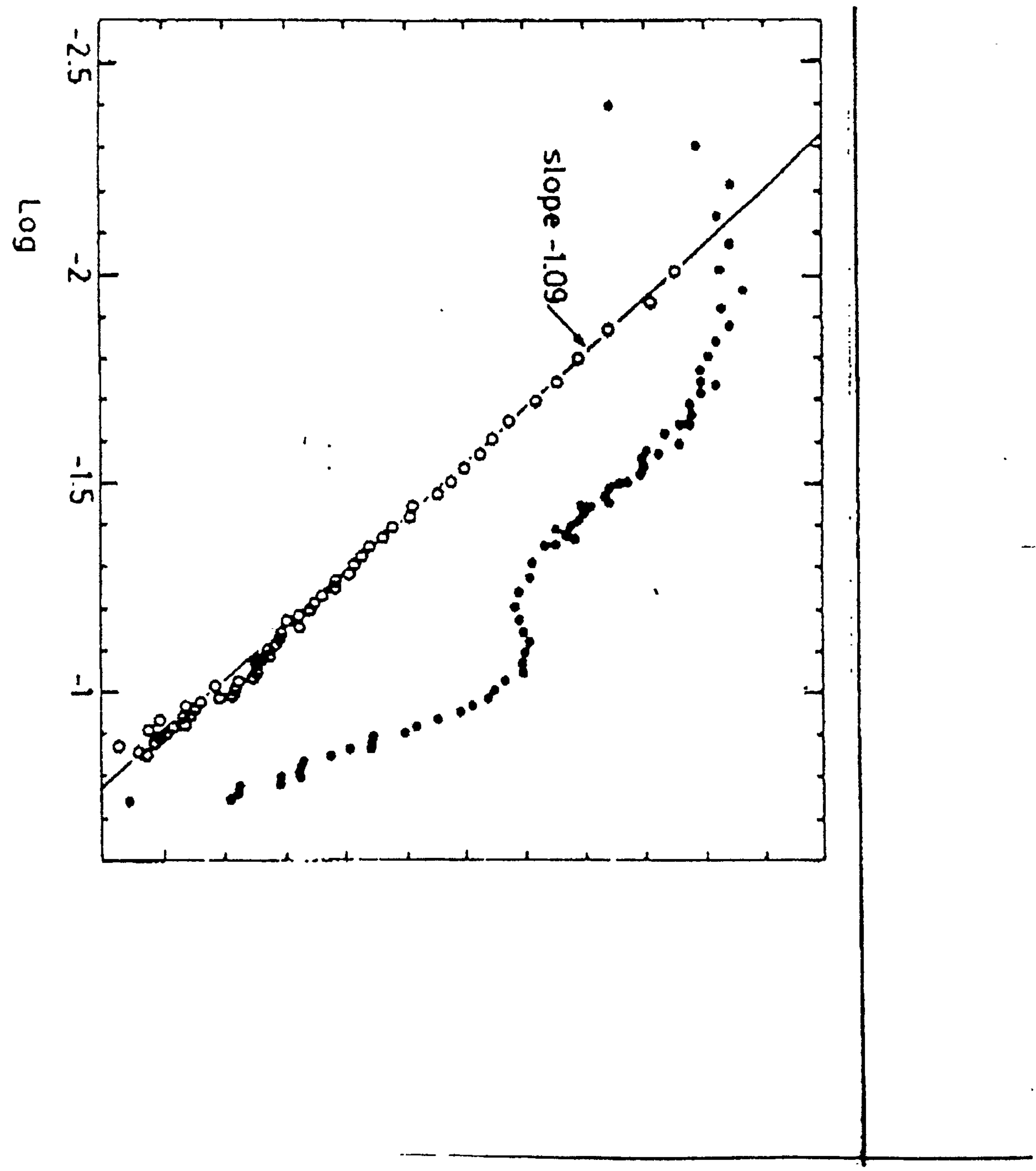


Streuungskurven von linearem Tyrocidin A o------o und Vasopressin •------• in PEG 1.500

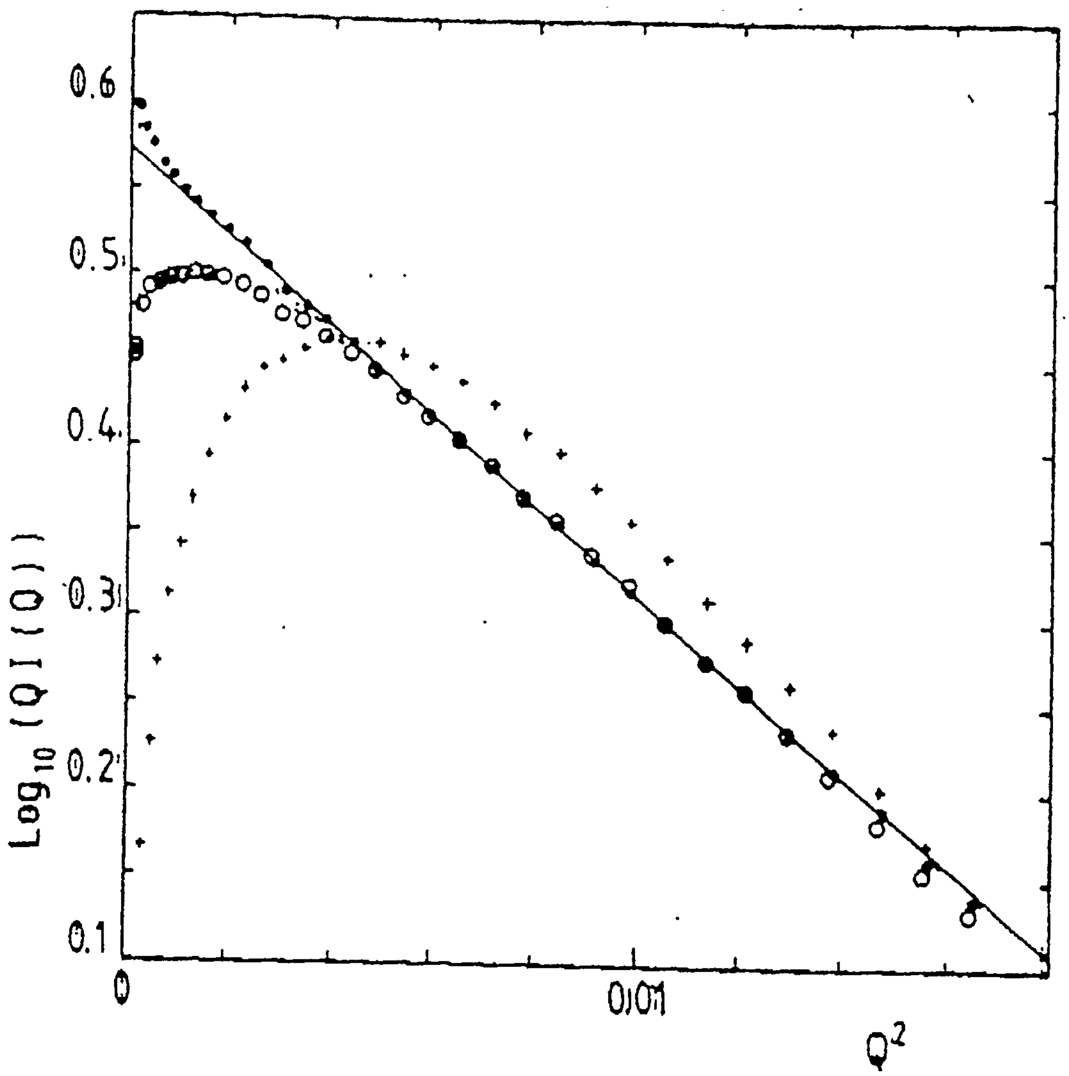


Abb. 3

Streuungskurven für S-(+)-Ibuprofen (o——o), Schmelze in einer PEG 1.500 der nicht zyklischen Form von Mofebutazon (x......x) und Dinoprost (•——•) beide an einer PEO 2000 Schmelze, nach Auflösen des Inhaltes einer Hartgelatinekapsel bei 37°C, in einer wäßrigen Lösung von 0.01 M $K_2HPO_4$ pH 5.5

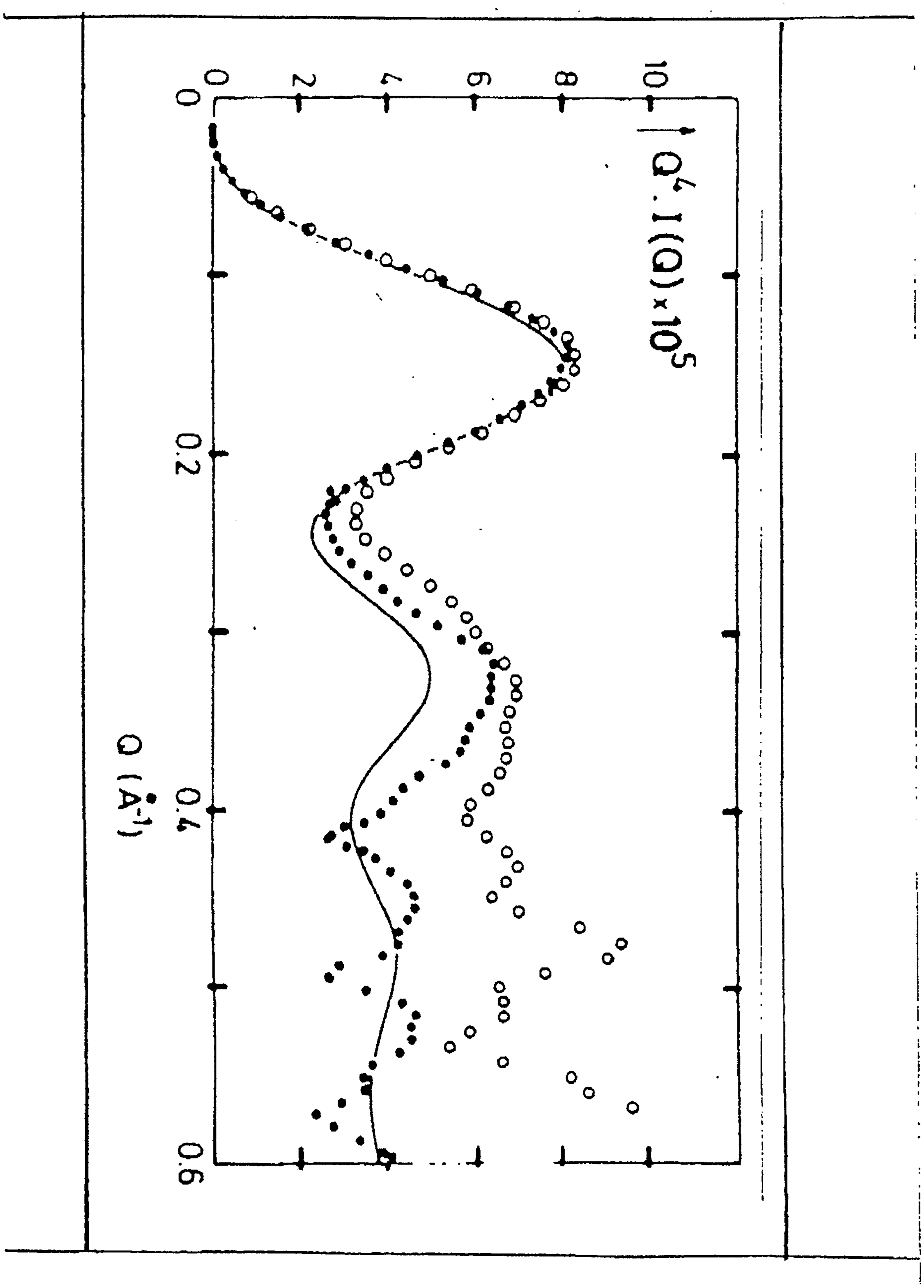


Abb. 4

Streukurven für S-ibuprofen entsprechend Fig. 3, jedoch bei höheren Streuwinkeln.

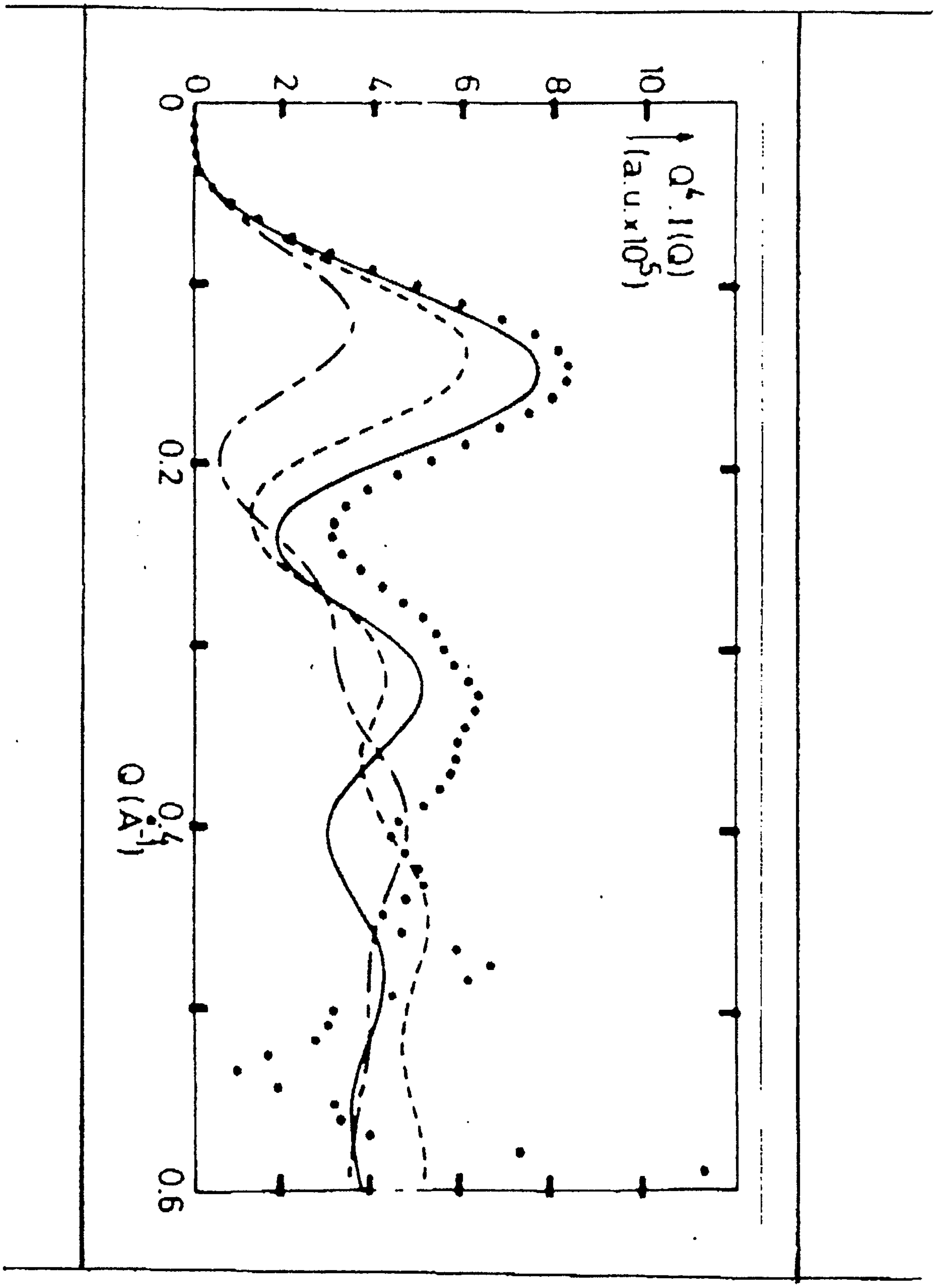


Abb. 5

Streukurven für S-ibuprofen entsprechend Fig. 3, jedoch für PEG 2500 und bei höheren Streuwinkeln.

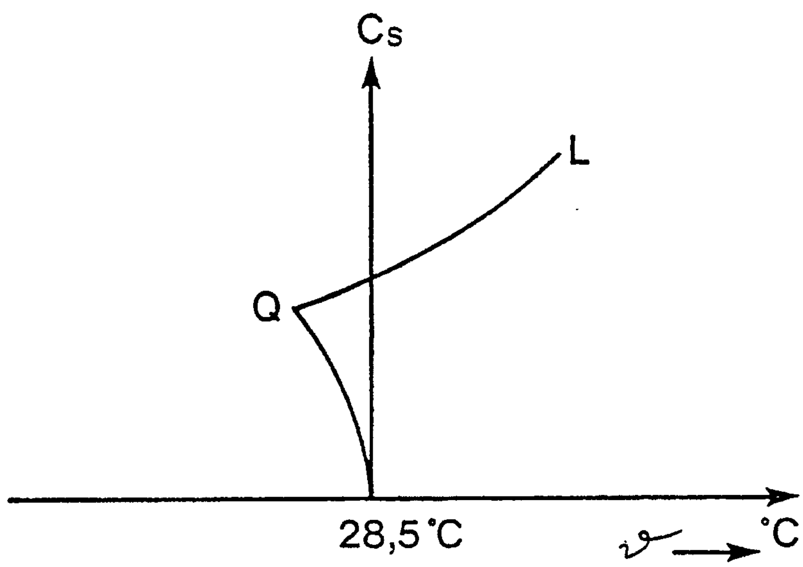


"Kyro-Solvat" ischer Punkt (Q) als gemeinsamer Endpunkt der Lösichkeitskurve L ⟶ Q un der Schelzkurve (Gefrierpunktserniedrigung) 28,5°C ⟶ Q im System (S)-Ibuprofen-Polyethylenoxid.

Abb. 6

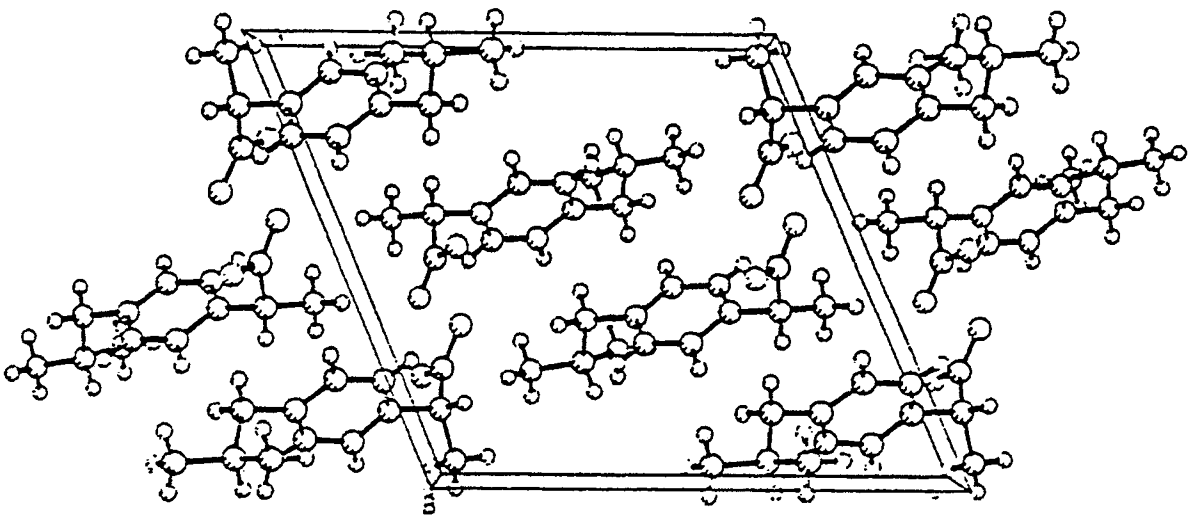

Kristallgitter (R)-Ibuprofen

Abbildung 7

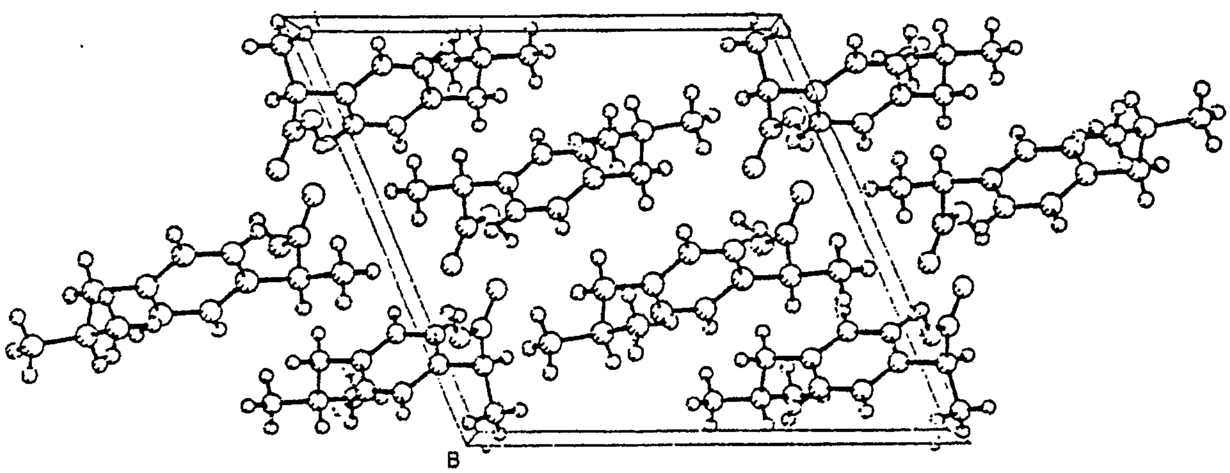

Kristallgitter (S)-Ibuprofen

Abbildung 8

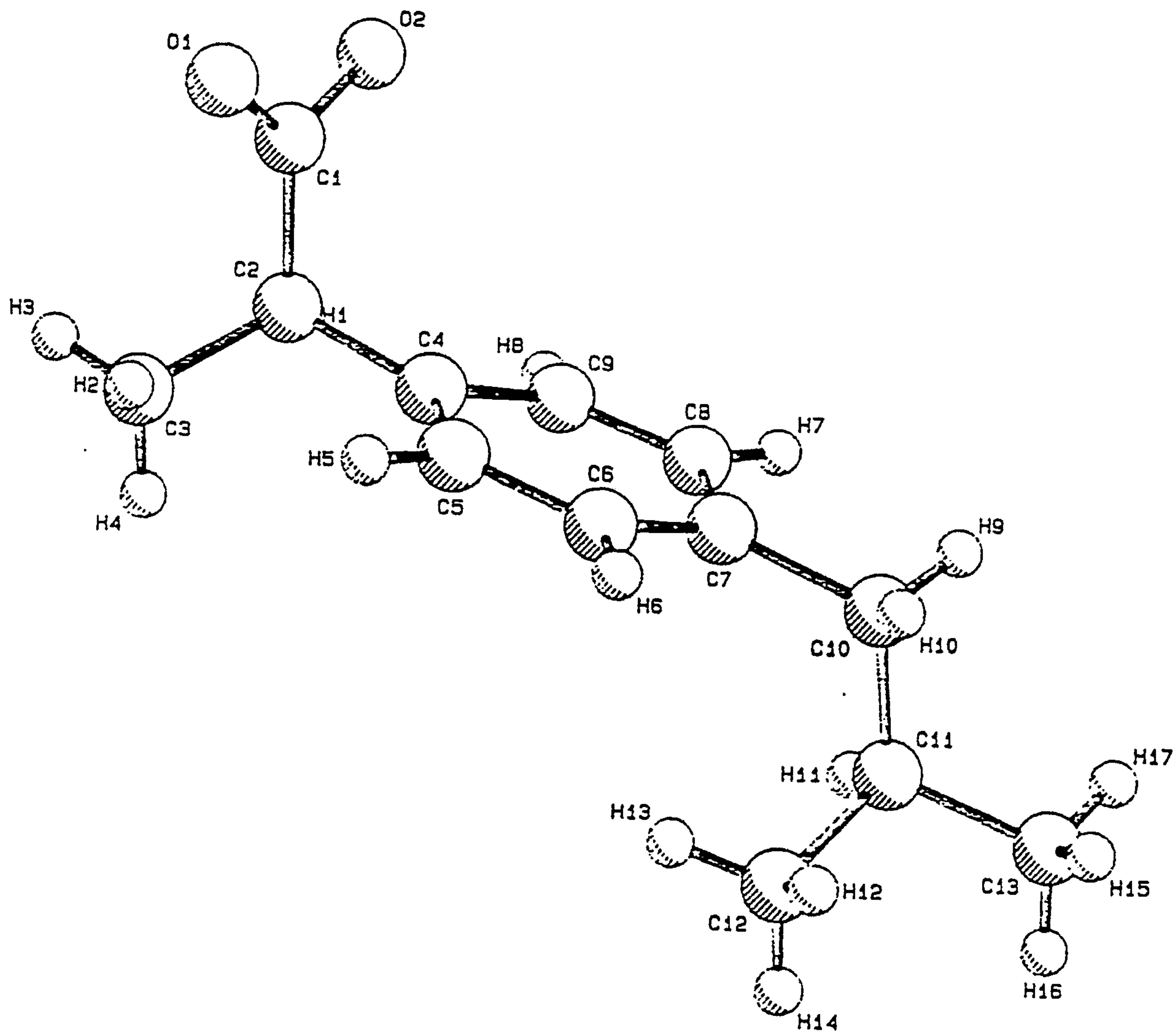

Molekulare Konformation von (R)-Ibuprofen

Abbildung 9

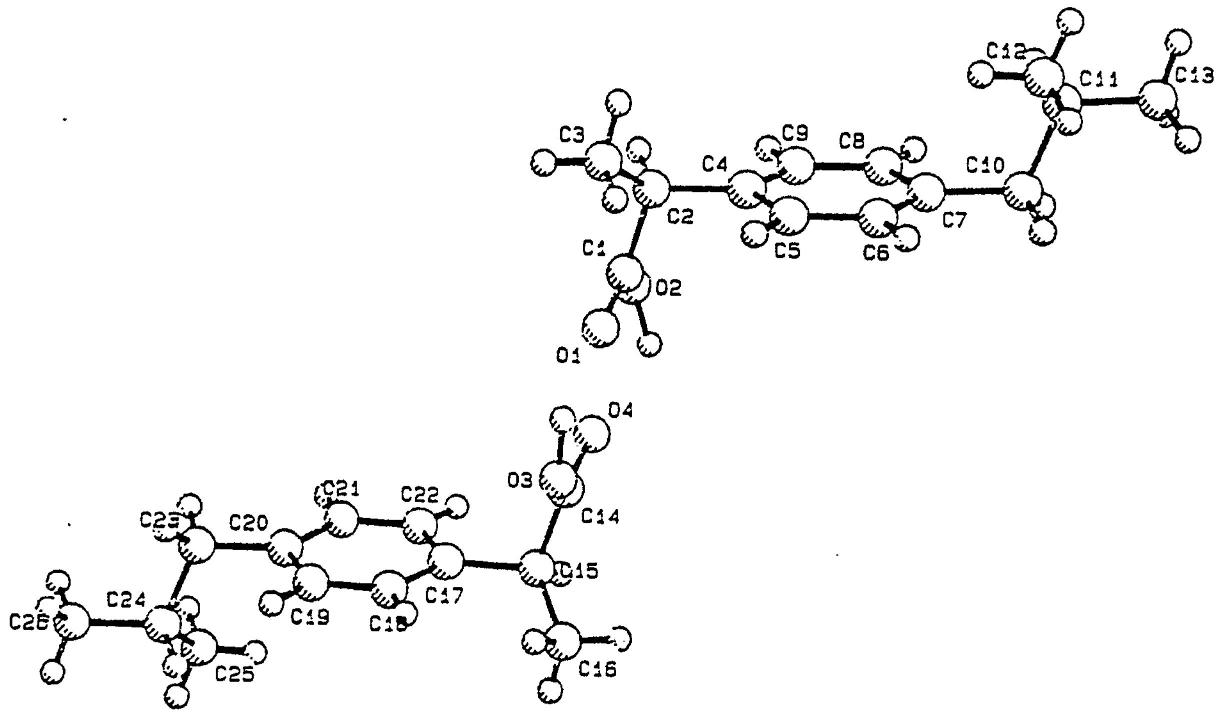

Molekulare Konformation von (S)-Ibuprofen

Abbildung 10

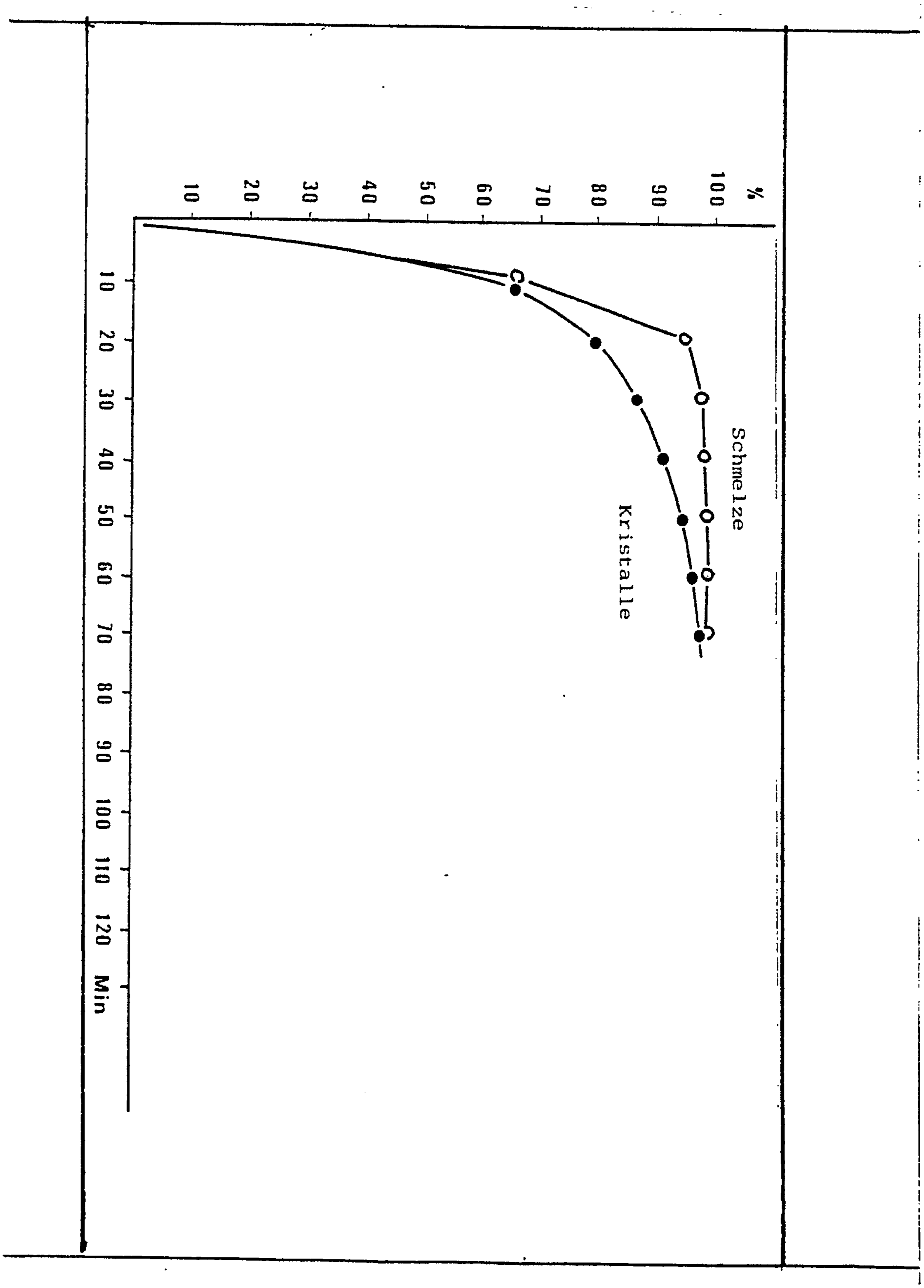


Abb. 11

In vitro-Freisetzung von S-ibuprofen (Kristalle und Schmelze).

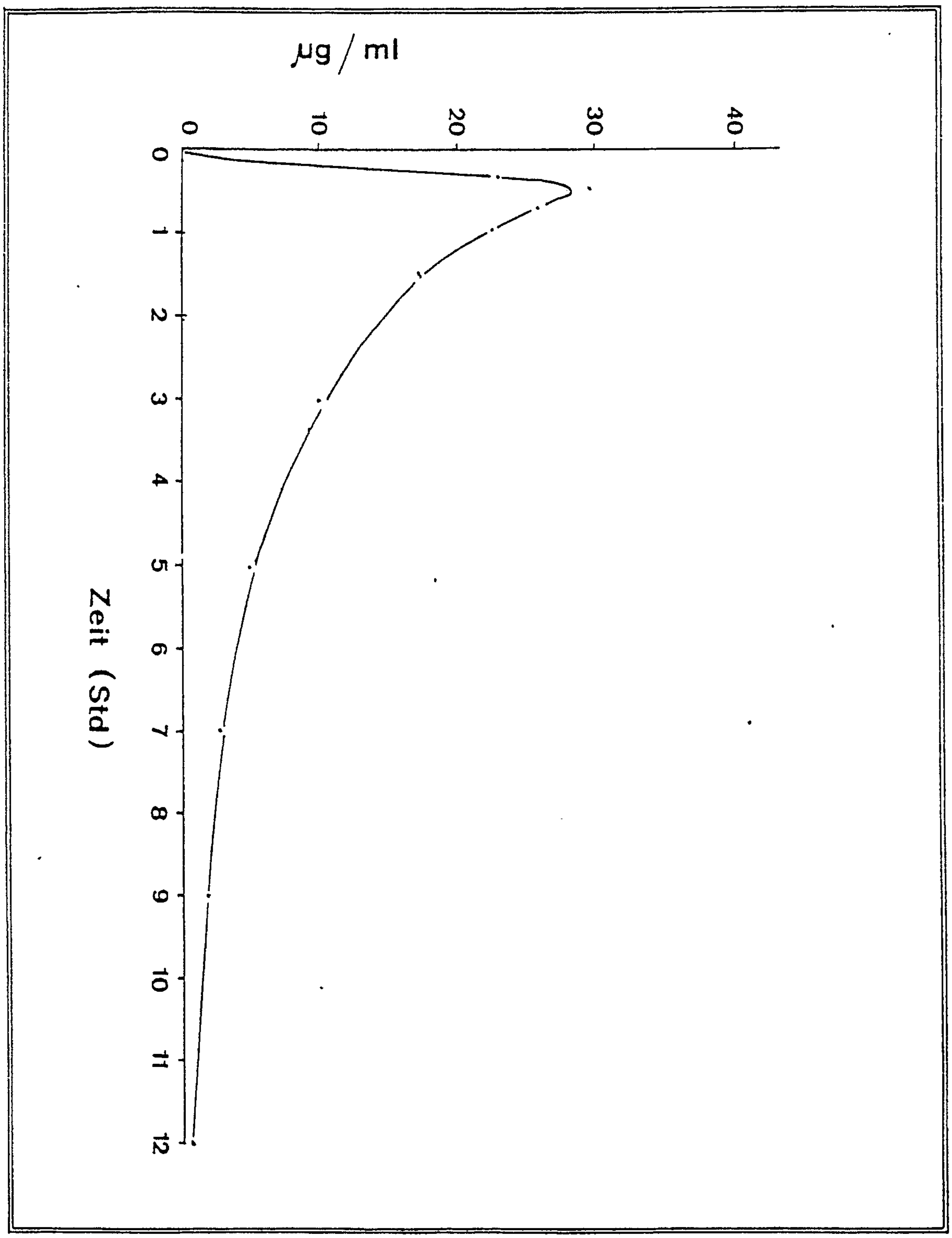


Abb. 12

In vivo-Freisetzung von S-ibuprofen (200 mg S-ibuprofen) Plasmaspiegel in µg/ml.

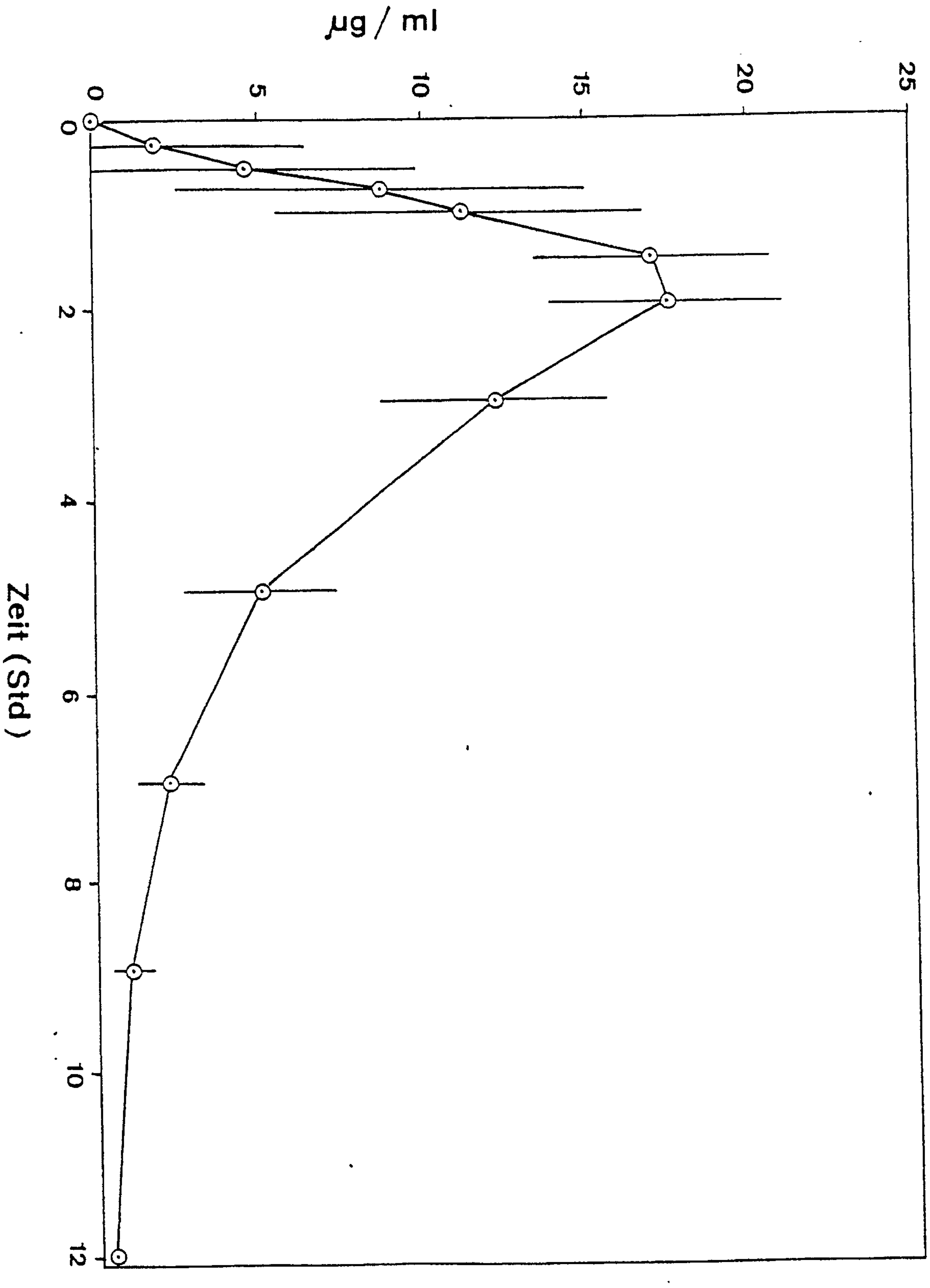


Abb. 13

Freisetzungsverhalten einer Tablette mit 400 mg S-ibuprofen

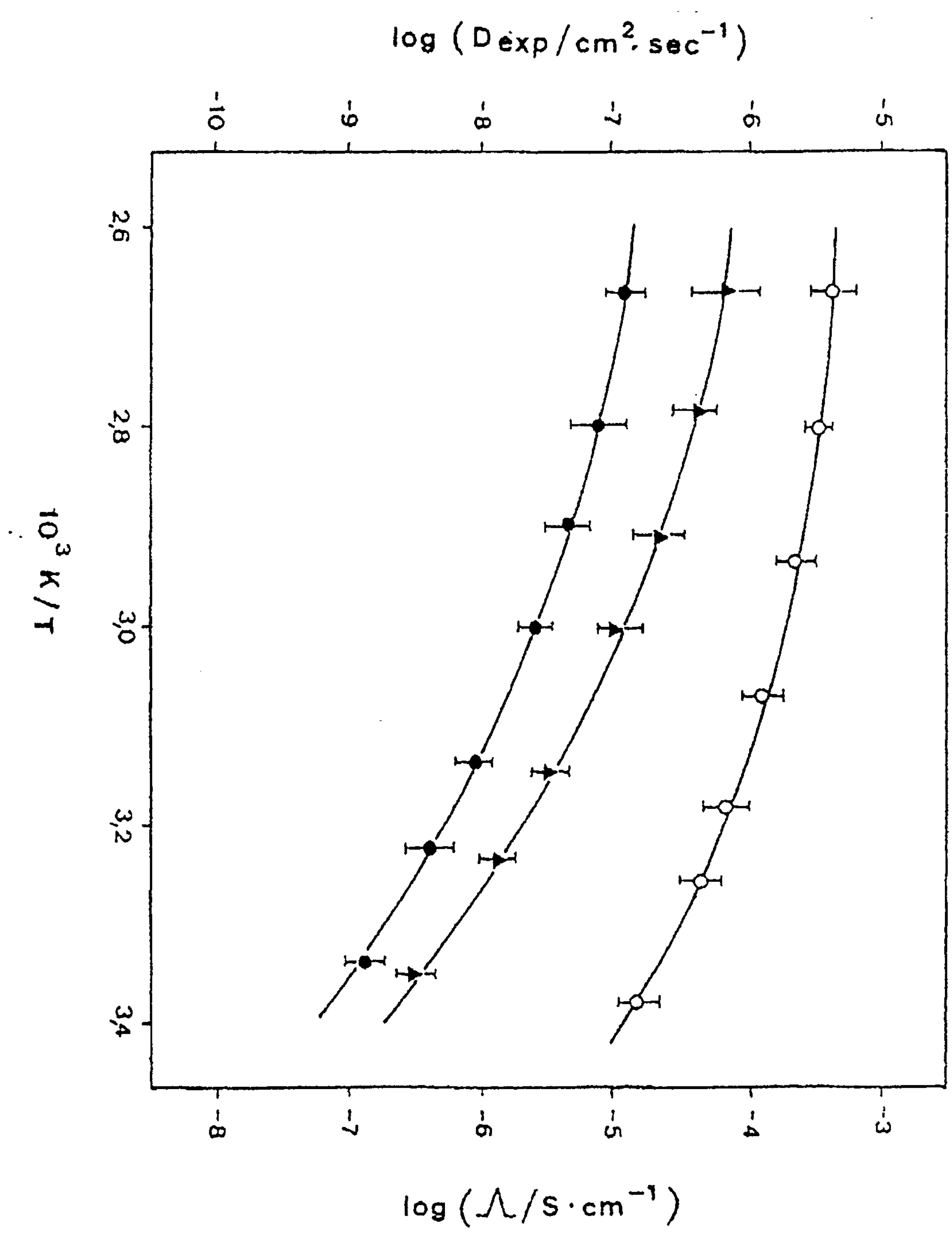


Abb. 14

Temperaturabhängigkeit des experimentellen Diffusionskoeffizienten ($D_{exp}$) und der elektrischen Leitfähigkeit (Λ) bei ●—● 100 mM S-(+)Ibuprofen in PEG; bei ▲—▲ 200 mM S-(+)Ibuprofen in PEG, sowie ○—○ 50 µg $PGE_2$ in PEG.

Europäisches Patentamt

# EUROPÄISCHER RECHERCHENBERICHT

Nummer der Anmeldung

EP 90 10 9234

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 182 007 (Dr. RENTSCHLER ARZNEIMITTEL GmbH & CO.)<br>* Insgesamt * | 1-6 | A 61 K 9/08<br>A 61 K 9/48 |
| Y |  | 1-6 | |
| A |  | 8 | |
| X | EP-A-0 299 668 (THE BOOTS CO., PLC)<br>* Seite 2, Zeilen 35-42; Seite 10, Beispiel 14 * | 1-6 | |
| Y | US-A-4 151 273 (RIEGELMAN et al.)<br>* Insgesamt * | 1-6 | |
| Y | US-A-3 862 311 (LEESON)<br>* Insgesamt * | 1-6 | |
| Y | GB-A- 942 743 (THE WELLCOME FOUND. LTD)<br>* Seite 2, Beispiel 1 * | 1-6 | |
| A | FR-A-1 591 573 (CENTRE DE RECHERCHES MARCEL MIDEY) | | **RECHERCHIERTE SACHGEBIETE (Int. Cl.5)** |
| D,A | EP-A-0 001 822 (HOECHST AG)<br>* Seite 4, Zeile 7 - Seite 6, Zeile 5; Seiten 11-13, Beispiele 1,3 * | 1-6 | A 61 K |
| T | JOURNAL OF PHARMACEUTICAL SCIENCES, Band 60, Nr. 9, September 1971, Seiten 1281-1302; W.L. CHIOU et al.: "Pharmaceutical applications of solid dispersion systems"<br>* Seite 1286, Spalte 2, "Solid solutions" - Seite 1293, Spalte 1, Zeile 26 * | 1-8 | |

Der vorliegende Recherchenbericht wurde für alle Patentansprüche erstellt

| Recherchenort | Abschlußdatum der Recherche | Prüfer |
|---|---|---|
| DEN HAAG | 17-08-1990 | BENZ K.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)